# EUROPEAN PATENT SPECIFICATION

(11) **EP 3 664 713 B1**
(45) Date of publication and mention of the grant of the patent: **07.10.2026**
(21) Application number: 18843590.3
(22) Date of filing: 10.08.2018
(51) Int. Cl.: A61B 6/04

(54) **BREAST COMPRESSION PADDLE WITH ACCESS CORNERS**
BRUSTKOMPRESSIONSPLATTE MIT ZUGANGSECKEN
PLAQUE DE COMPRESSION DU SEIN POURVUE DE COINS D'ACCÈS

(30) Priority: 11.08.2017 US 201762544615 P
(43) Date of publication of application: 17.06.2020
(73) Proprietor: Hologic, Inc., Marlborough, MA 01752 (US)
(72) Inventor: BANKS, Melissa, Marlborough Massachusetts 01752 (US); BERKO, Mallory, Marlborough Massachusetts 01752 (US); CABLE, Frank, Marlborough Massachusetts 01752 (US); PATEL, Tarpit, Marlborough Massachusetts 01752 (US); DEFREITAS, Kenneth F., Marlborough Massachusetts 01752 (US); STANGO, Timothy R., Marlborough Massachusetts 01752 (US)
(74) Representative: Carpmaels & Ransford LLP
(86) International application number: PCT/US2018/046312
(87) International publication number: WO 2019/033029

(56) References cited:
- WO-A1-2010/102087
- DE-A1- 102017 207 147
- KR-A- 20140 058 066
- US-A- 4 943 986
- US-A- 5 398 272
- US-A1- 2014 296 701
- US-A1- 2015 282 770
- US-A1- 2016 081 633
- US-A1- 2016 081 633

## Description

With reference to DE 10 2017 207 147 A1 in Germany, the applicant has voluntarily limited the scope of the patent for Germany by submitting separate claims for this state.

### Introduction

A significant concern in x-ray rnamrnography and breast tomosynthesis is the discomfort the patient may feel when the breast is compressed, typically, between two rigid plastic surfaces, with sufficient force to immobilize the breast and spread out the breast tissues for x-ray imaging. One challenge is to ensure that the imaged field includes the desired amount of breast tissue. The reasons for using compression include: ( I) to make the breast thinner in the direction of x-ray flux and thereby reduce patient radiation exposure from the level required to image the thicker parts of a breast that is not compressed; (2) to make the breast more uniform in thickness in the direction of x-ray flux and thereby facilitate more uniform exposure at the image piane over the entire breast image; (3) to immobilize the breast during the x-ray exposure and thereby reduce image blurring; and (4) to bring breast tissues out from the chest wali into the imaging exposure field and thus image more tissue. As the breast is being compressed, typically a technician manipulates the breast to position it appropriately and counter the tendency that compression has of pushing breast tissue toward the chest wall and out of the image field.

Standard compression methods for mammography and tomosynthesis use a movable, rigid elear plastic compression paddle in which the surfaces of the paddle are perpendicular to one another. The breast is placed on a breast platform that typically is fiat, and the paddle is then compressed onto the breast, usually while a technician or other health professional is holding the breast in place and perhaps manipulates the breast to ensure proper tissue coverage in the image receptor's field of view and to help spread the breast. However, the size and shape of the paddle often prevent the technician from properly holding and manipulating the breast. That is, the technician's hand may get stuck between the compression paddle and breast as it is lowered, which can cause stress to both the patient and the technician. Altematively, the technician may release the breast too soon in an effort to avoid getting stuck, resulting in undesirable compression. Both circumstances may potentially increase the length of the procedure, adversely impact patient positioning, and affect image quality.

Document US 5,398,272 discloses a compression device for a radiological apparatus, the compression device including a compression pad and fastening structure to connect the compression pad to driving elements that are fitted onto the radiological apparatus, wherein both the compression pad and the fastening means are a single unitary piece of injection molded plastic that is substantially transparent to X-rays.

### Summary

The present invention relates to a breast compression paddle for an imaging system, the breast compression paddle having: a plurality of boundary walls having a front boundary wall, a rear boundary wall, and two lateral boundary walls extending from the front boundary wall to the rear boundary wall; a bracket portion extending from the rear boundary wall away from the front boundary wall; and a bottom wall and a transition wall connected to each of the plurality of boundary walls at a curved interface, wherein the bottom wall includes at least three points defining a bottom reference plane, and wherein an uppermost portion of the front boundary wall is disposed at a front boundary wall height above the bottom reference plane, and wherein an uppermost portion of the rear boundary wall is disposed at a rear boundary wall height above the bottom reference plane, and wherein the front boundary wall height is less than the rear boundary wall height. The bracket portion is raised relative to a compression portion of the paddle so as to define an access area below at least a portion of the compression paddle, and the transition wall extends from the bottom wall towards the bracket portion and at least partially defines the access area. In an embodiment, the bottom wall includes a raised central portion and a plurality of outer edge portions, and wherein the at least three points are disposed on the plurality of outer edge portions. In another embodiment, the bottom wall includes two outer edge portions disposed proximate the two lateral boundary walls, and a central portion substantially level with the two outer edge portions. In yet another embodiment, the bottom wall includes a proximate portion disposed proximate to the front boundary wall and a distal portion disposed distal from the front boundary wall and wherein the distal portion is disposed a distal portion distance above the bottom reference plane. In still another embodiment, the bracket portion is connected to the rear boundary wall and includes a rear paddle wall disposed opposite from the rear boundary wall and disposed substantially parallel to the rear boundary wall.

In another embodiment, the breast compression paddle further having a plurality of ribs spanning from the rear boundary wall to the rear paddle wall. In an embodiment, at least one of the plurality of ribs contacts at least a portion of the bottom wall. In another embodiment, at least a portion of the transition wall is convex. In yet another embodiment, the front boundary wall defines a recess having an upper width disposed proximate the uppermost portion of the front boundary wall and a lower width disposed proximate the bottom wall, wherein the lower width is greater than the upper width. In still another embodiment, the recess is disposed at an angle to the front boundary wall.

In yet another embodiment, the compression paddle utilizes smooth contours on all surfaces in the imaging area so as to reduce or prevent such artifacts. In still another embodiment, the compression paddle comprises ribs that extend to a location on the transition wall that is lower than the front boundary wall height. In a further embodiment, the compression paddle comprises an open, bowl-like configuration. In yet another embodiment, the transition wall has a generally smooth curvature that connects the bottom wall and a bottom surface of the bracket portion.

### Brief Description of the Drawings

The same number represents the same element or same type of element in all drawings.
FIG. 1 is a schematic view of a mammography and/or tomosynthesis system.
FIG. 2 is a partial perspective view of a mammography and/or tomosynthesis system.
FIG. 3 is an upper perspective view of a compression paddle and bracket.
FIGS. 3A and 3B are upper and lower perspective views of a compression paddle.
FIGS. 3C-3H are various views of the compression paddle of FIGS. 3A and 3B.
FIGS. 4A-4E are cross sectional views of the compression paddle of FIG. 3E.
FIGS. 5A-5C are perspective, front, and side views, respectively, of another example of a compression paddle.
FIGS. 6A-6C are perspective, front, and side views, respectively, of another example of a compression paddle.
FIGS. 7A-7C are perspective, front, and side views, respectively, of another example of a compression paddle.
FIG. 8 is a rear perspective view of another example of a compression paddle.
FIGS. 9A-9C depict a method of using a compression paddle.

### Detailed Description

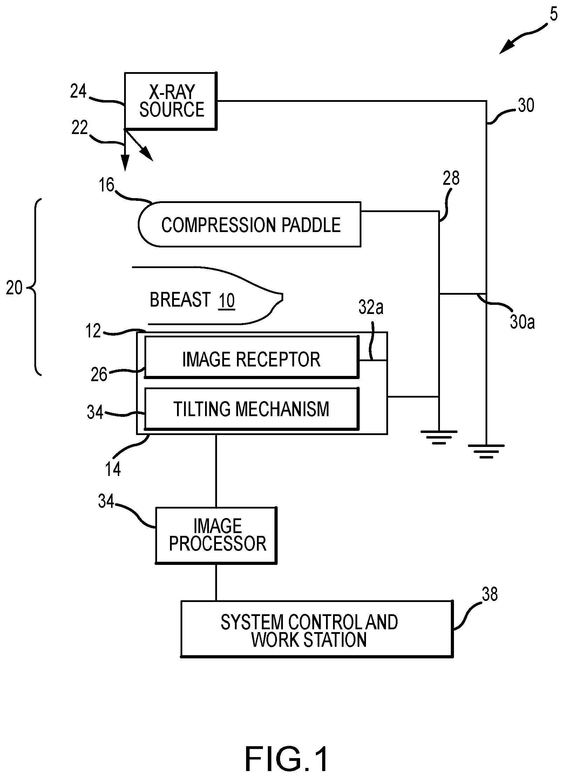

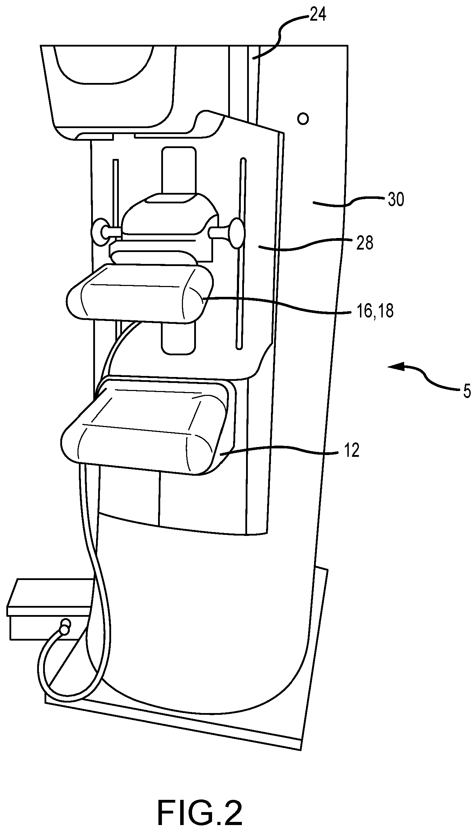

FIG. 1 is a schematic view of a mammography and/or tomosynthesis system 5 using a compression paddle in imaging a patient's breast with x-rays. FIG. 2 illustrates in perspective view a system 5 in which components are identified by reference numeral used in FIG. 1 and described in concurrently with FIG. 1. A patient's breast 10 is immobilized for x-ray imaging between a breast platform 12 and a compression paddle 16. Platform 12 can be the upper surface of a housing 14. Platform 12 and paddle 16 form a breast immobilizer unit 20 that is in a path of an imaging beam 22 emanating from x-ray source 24. Beam 22 impinges on image receptor 26 that is in housing 14.

Immobilizer 20 and housing 14 are supported on an arm 28. X-ray source 24 is supported on an arm 30. For mammography, support arms 28 and 30 can rotate as a unit about an axis such as at 30a between different imaging orientations such as CC and MLO, so that the system 5 can take a mammogram projection image Mp at each orientation. Image receptor 26 remains in place relative to housing 14 while an image Mp is taken. Immobilizer 20 releases breast 10 for movement of arms 28 and 30 to a different imaging orientation. For tomosynthesis, support arm 28 stays in place, with breast 10 immobilized and remaining in place, while at least source support arm 30 rotates source 24 relative to immobilizer 20 and breast 10 about an axis such as 30a.

The system takes plural tomosynthesis projection images of breast 10 at respective angles of beam 22 relative to breast 10. Concurrently, image receptor 26 may be tilted relative to breast platform 12 in sync with the rotation of source support arm 30. The tilting can be through the same angle as the rotation of course 24, but preferably is through a different angle, selected such that beam 22 remains substantially in the same position on image receptor 26 for each of the plural images Tp. The tilting can be about an axis 32a, which can but need not be in the image plane of image receptor 26.

In one embodiment, optionally, a tilting mechanism 34, which also is in housing 14 or is otherwise coupled with receptor 24, can drive image receptor 24 in a tilting motion. Axes 20a, 24a and 26a extend left-right as seen in FIG. 1, and may but preferably do not coincide. For tomosynthesis imaging, breast platform 12 can be horizontal or can be at an angle to the horizontal, e.g., at an orientation similar to that for conventional MLO imaging in mammography. The system of FIG. 1 can be solely a mammography system, or solely a tomosynthesis system, or a "combo" system that can perform both mammography and tomosynthesis imaging. An example of such a combo system is been offered by the assignee hereof under the trade name Selenia Dimensions. Nonlimiting examples of such a combo system or a tomosynthesis system are described at U.S. Patent Nos. 7,869,563; 7,831,296; 7,583,786; 7,430,272; 7,245,694; and 7,123,684. When the system is operated, image receptor 26 produces imaging information in response to illumination by imaging beam 22, and supplies it to image processor 34 for processing to generate breast x-ray images. Given the complex shapes of the compression paddles described herein, the image processor may utilize image processing functionality as described in co-owned PCT Publication No. WO 2018/089118, filed September 25, 2017, entitled "Imaging with Curved Compression Elements," A system control and work station unit 38 controls the operation of the system and interacts with a user to receive commands and deliver information including processed-ray images.

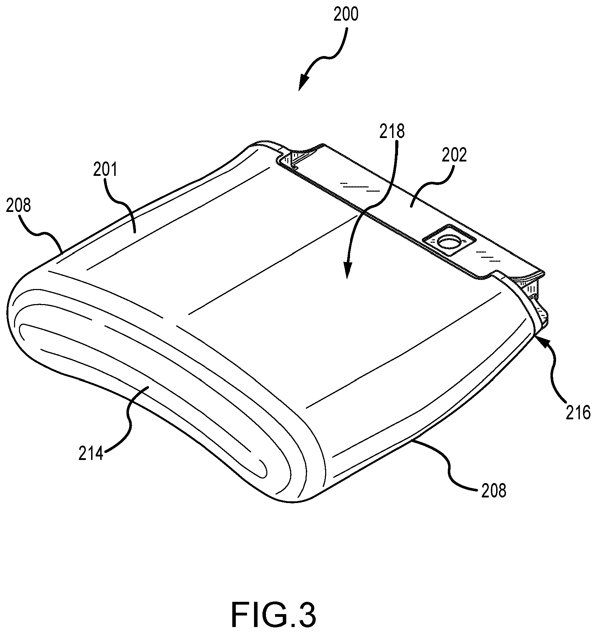

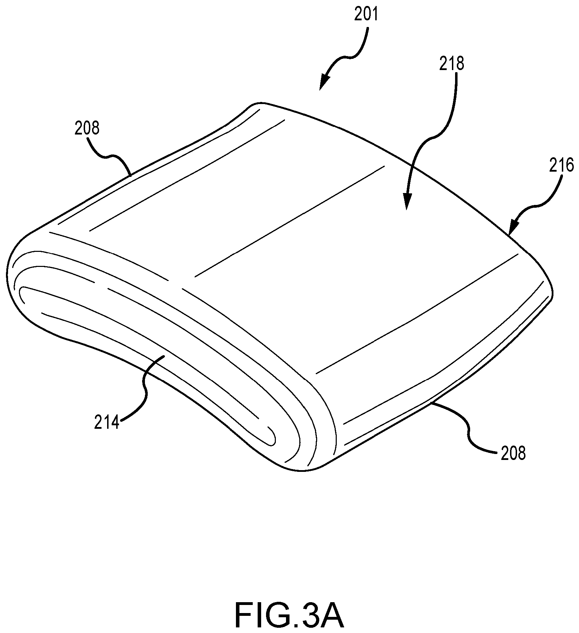

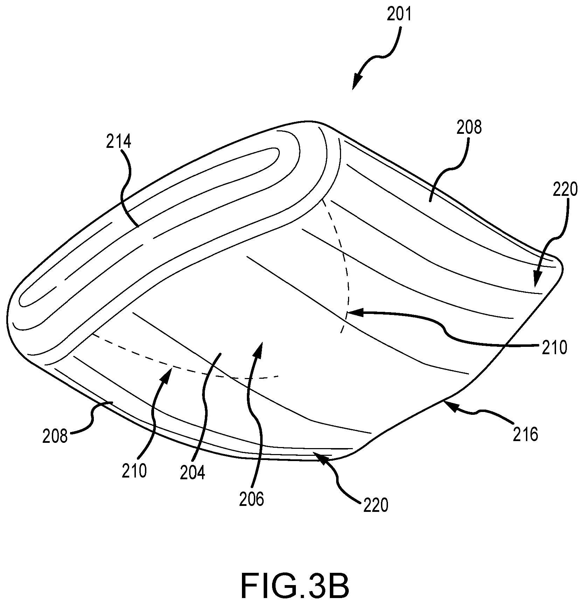

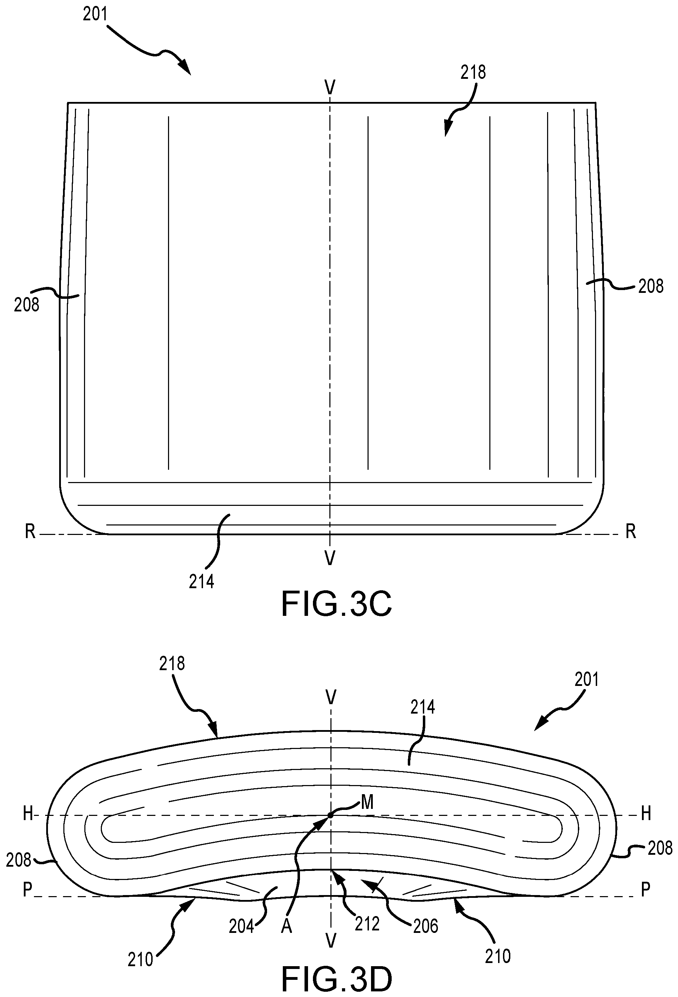

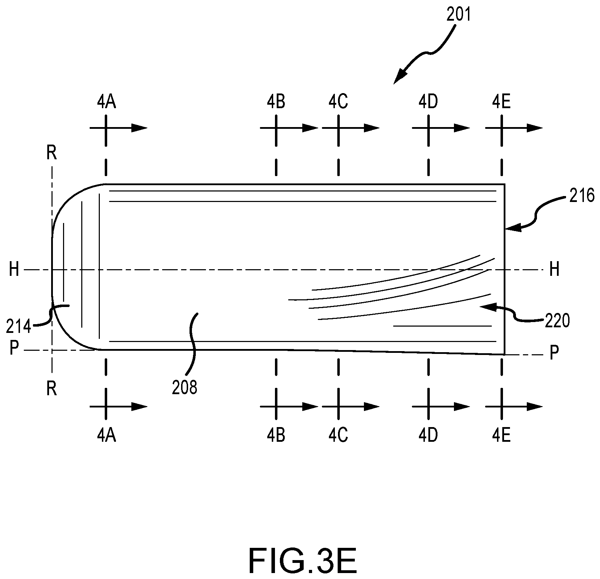

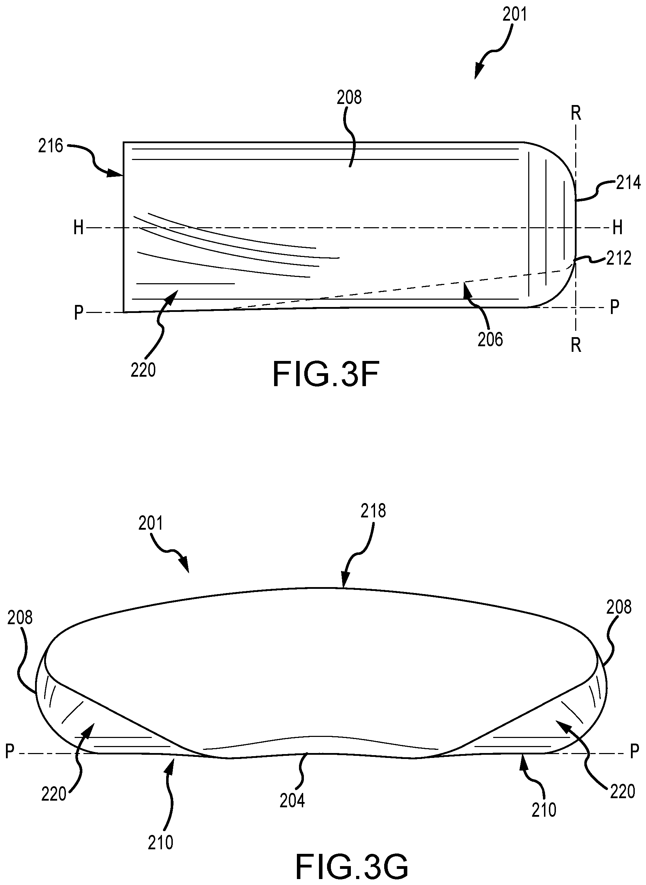

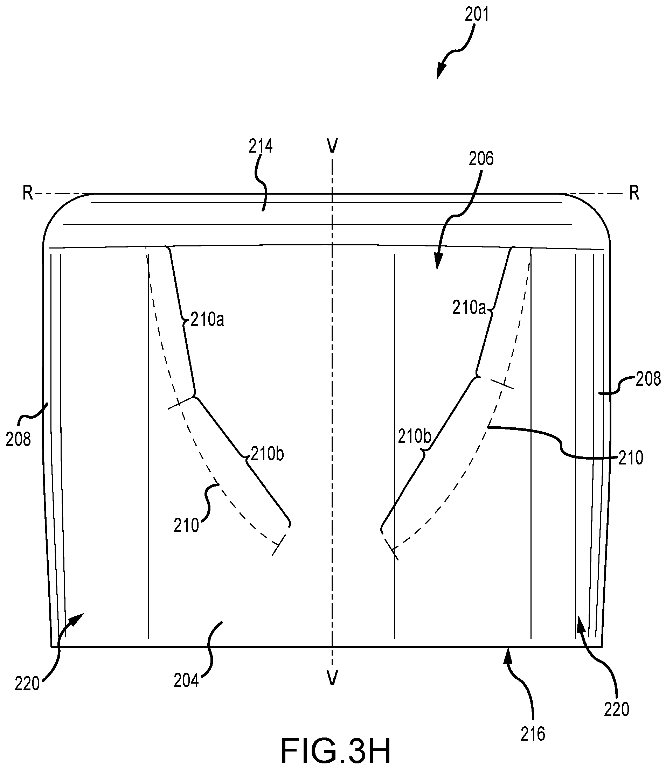

FIG. 3 is an upper perspective view of a compression element 200 including a compression paddle 201 and bracket 202, while FIGS. 3A and 3B are upper and lower perspective views of the compression paddle 201. FIGS. 3C-3H are various views of the compression paddle 201 of FIGS. 3A and 3B. FIGS. 3-3H are described concurrently. The compression paddle 201 compresses a breast of a patient during imaging procedures. The bracket 202 removably connects the compression element 200 to an arm of the imaging system. The depicted compression paddle 201 is configured to allow a technician to have improved access to a patient breast during lowering of the compression paddle 201. This operation is described in more detail below. The compression paddle 201 includes a number of features that enable such access.

A bottom wall 204 of the compression paddle 201 includes a generally concave surface 206, which generally may correspond in shape to a breast and/or a compressed breast. The generally concave surface 206 can extend generally between the rounded side walls 208 of the compression paddle 201. Alternatively, only a portion of the bottom surface includes a generally concave surface, which helps to match the contour of breast tissue. The generally concave surface 206 helps to distribute more equally forces applied to the breast to more closely correspond to the shape of the breast. Such a configuration may help provide more comfort to a patient as the breast is being compressed. The generally concave surface 206 is defined by an outer edge 210 (depicted by dashed lines in FIGS. 3B and 3H) that defines a bottom reference plane P, as well as a central portion 212. In another example, the lowest part of the compression paddle 201 (that is, the portions on which the compression paddle 201 may rest when placed on a flat surface) may define the bottom reference plane P. In the depicted example, the shape of the outer edge 210 changes as distance from a front wall 214 of the compression paddle 201 increases. As can be seen, proximate the front wall 214, the outer edges 210 are more linear 210a in configuration, while further therefrom, the shape is more curved 210b. In another example, the outer edges 210 may be linear in configuration along their entire lengths. This is to mimic the approximate outer shape of a breast. The central portion 212 is non-coplanar with the outer edge 210, such that the central portion 212 is raised relative to or disposed above the bottom reference plane P. The central portion 212 may be level (e.g., parallel to the bottom reference plane P of the paddle 201) or may be pitched downward from the rounded front wall 214 to a rear edge 216 (where the paddle 201 connects to the bracket 202). The portion of the rounded front wall 214 nearest to the patient during imaging procedures may be referred to as the front reference surface. The pitch of central portion 212 is depicted specifically in FIG. 3F and may help further conform the paddle 201 to the shape of the breast.

The generally concave surface 206 may also help to prevent the breast from slipping and moving during positioning of the breast, as well as during image acquisition. As an example, this configuration may help prevent slipping of the breast in the MLO position by supporting the breast more, in comparison to known flat compression paddles that often allow the breast to slip during image acquisition. The generally concave surface 206 may have a smooth curvature or can have any other shape that is generally concave, e.g., the surface 206 may include ridges, lines, and/or other elements from injection molding the compression paddle 201, the surface may have a generally trapezoidal shape, etc. Additionally or alternatively, the compression paddle 201 can be used to compress a patient's breast with or without an inflatable jacket and/or a gel pad. In another example, the generally concave surface 206 may not be uniformly concave from the front wall 214 (i.e., the chest facing wall) to the rear edge 216. As compressed breast tissue may not extend as far back as the rear edge 216, the concavity may be greater near the front wall 214 compared to the rear edge 216. As an example, the bottom wall 204 may be generally concave near the front wall 214 and may be flatter near the rear edge 216. In an additional or alternative example, the radius of the generally concave surface 206 is greater near the front wall 214 compared to the bottom wall 204 near the rear edge 216. This nonuniformity may help to provide more even compression from the nipple to the chest wall of the breast.

Generally, the compression paddles of the present technology described herein may be more comfortable to a patient undergoing breast compression during a mammogram or other x-ray imaging of the breast, as well as provide greater technician access to the breast for proper patient positioning. Positioning of the breast is important to produce images that are be diagnostically useful for radiologists. The compression paddles of the present technology described herein generally require less compression force to be applied to accomplish the same tautness as that of a known flat compression paddle. The paddles may be manufactured of substantially rigid or flexible materials. Use of rigid materials allows the paddle to sufficiently compress the breast without undesirably deforming. For example, a distance between the central portion 212 and the plane P may be substantially the same when the paddle 200 is compressing a breast or not compressing a breast. For example, the concave contour of the concave surface 206 may be substantially the same. The particular shapes and contours disclosed herein may reduce or eliminate discomfort during breast compression, and allow for increased access to the breast, as described further below.

The two rounded side walls 208 transition to a curved top surface 218 of the compression paddle 201, although in certain examples, a curved top surface 218 is not utilized and the paddle may be substantially bowl- or plate-shaped, with upper edges of the front wall 219 and rounded side walls 208 terminating at a location proximate where the top surface 218 is depicted. Notably, proximate the rear edge 216 of the compression paddle 201, the bottom wall 204 and rounded side walls 208 transition into access surfaces 220. The access surfaces 220 define an absence of each of the bottom wall 204 and rounded side walls 208. This absence allows for improved access of the technician to the patient breast, for example, when the compression paddle 201 is disposed proximate the breast, or once compression of the breast has begun. The transitions between various adjacent surfaces of the compression paddle 201 are smooth so as to limit patient discomfort and reduce stress locations in the compression paddle 201.

Two additional reference planes are also depicted in FIGS. 3C, 3D, 3E, 3F, and 3H, for clarity. These include a vertical reference plane V and a horizontal reference plane H. Both the vertical reference plane V and the horizontal reference plane H pass through a midpoint M on the curved front wall 214 of the compression paddle 201. The midpoint M is disposed along the axis A of the compression paddle 201, which is defined by the intersection of the vertical reference plane V and the horizontal reference plane H. The compression paddle 201 is symmetrical on either side of the vertical reference plane V. When utilized in an imaging system, the vertical reference plane V is disposed at an angle substantially orthogonal to the breast platform (not shown), while the horizontal reference plane H is disposed substantially parallel to the breast platform.

The complex contours of the compression paddle 201 enable it to comfortably compress the breast, while still allowing technician access to the breast during compression procedures. This allows the technician to manipulate or position the breast as required or desired. During a compression procedure, as well as during subsequent imaging, the front wall 214 is adjacent to and faces a chest wall of a patient. The portion of the front wall 214 closest to the patient chest wall is referred to as a front reference surface. A plane substantially tangential to this front reference surface is referred to as a front reference plane R and is substantially orthogonal to each of vertical reference plane V, horizontal reference plane H, and bottom reference plane B. The front wall 214 includes a curved lower interface that interfaces with the bottom wall 204. This curved lower interface has an upper extent, e.g., the highest location of the curved lower interface above the bottom reference plane P, the curved surfaces of which increase comfort to the patient during compression. The front reference surface is disposed tangential to this upper extent to further increase comfort. The two outer edge portions 210 define the bottom reference plane P. The bottom wall 204 extends away from the chest wall and is adjacent to a length of a top surface of the compressed breast. As described above, the bottom wall 204 is not flat, but instead includes a central portion 206 that is non-coplanar with the two outer edge portions 210. As depicted in FIG. 3F, a height of the central portion 206 over the bottom reference plane P decreases as a distance away from the front wall 214 increases patient comfort. The edge portions 210 extend away from the front reference plane R and front wall 214. The contours of the compression paddle 201 may also be defined relative to a number of reference planes depicted and described herein. For example, the vertical reference plane V is substantially orthogonal to each of the front reference plane R and the bottom reference plane P. The compression paddle 201 includes a plurality of access surfaces 220 that allow a technician to have improved access to the breast during compression procedures. The shapes, sizes, and configurations of prior art compression paddles often limit technician access to the breast. At best, this would cause improper positioning of the breast during compression, requiring removal of the compression force, attempted repositioning of the breast, and recompression. This can greatly increase procedure time as well as patient anxiety and discomfort. At worst, the technician's hand would get stuck between the breast and the compression paddle. The incorporation of one or more access surfaces 220 on either side of the compression paddle 201 reduces or entirely obviates these problems. The access surfaces 220 are disposed proximate the two outer edge portions 210, namely the curved portions distal the front wall 214, and are defined by a substantially or entirely flat access reference surface. The configuration of the access surfaces 220 is such that they are disposed at non-orthogonal angles to each of the front reference plane R, the bottom reference plane P, and the vertical paddle plane V.

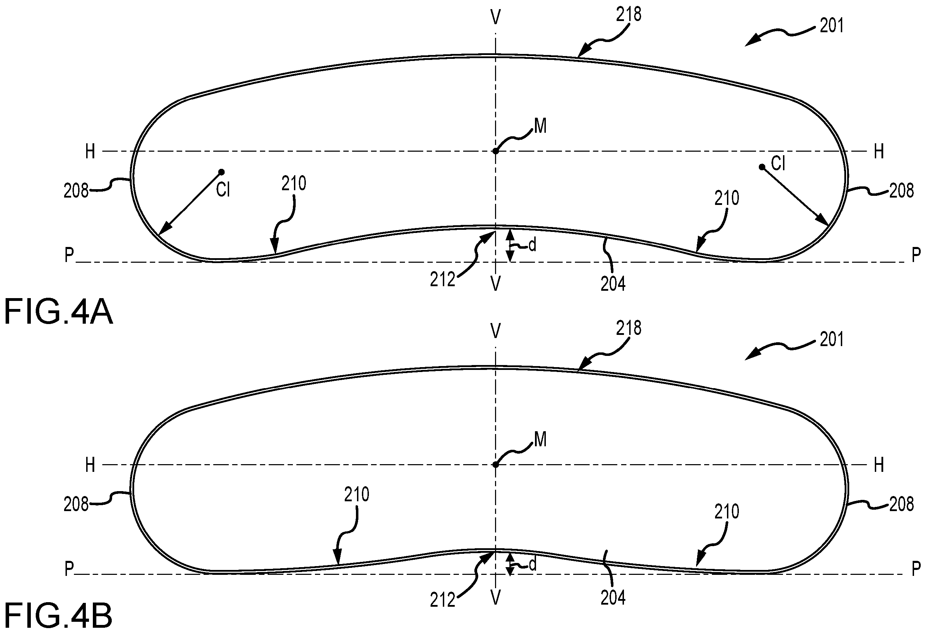

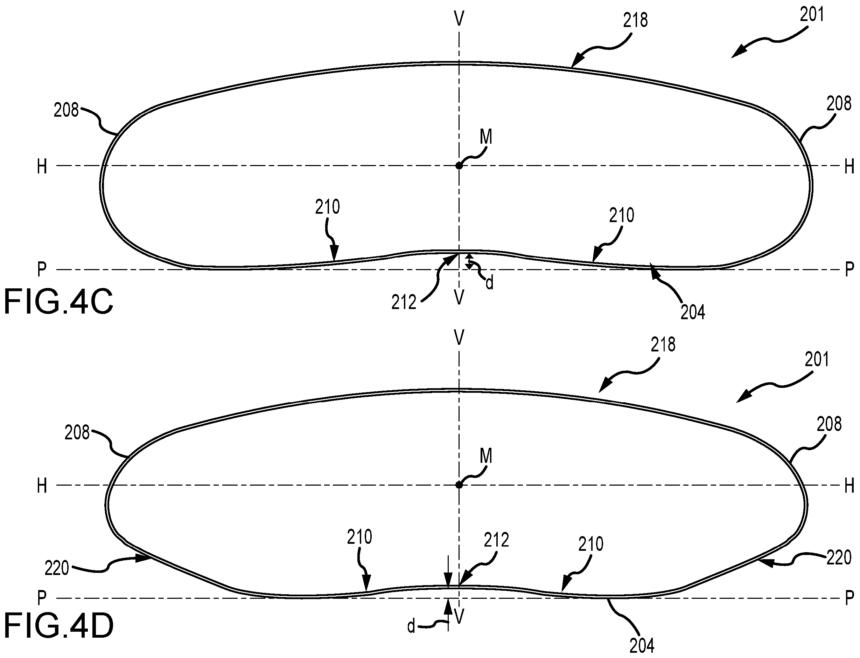

As apparent from FIGS. 3-3H, the compression paddle 201 described herein defines a changing cross section as distance from the front wall 214 increases. These changing cross-sections are further depicted in FIGS. 4A-4E, which depict cross sections of the compression paddle 201 as distance from the front wall 214 increases. The locations of the particular cross sections are depicted in FIG. 3E. In each of FIGS. 4A-4E, the vertical reference plane V, the horizontal reference plane H, and the bottom reference plane P are also depicted. The midpoint M of the front wall 214 is also depicted, and is positioned at the intersection of the vertical reference plane V and the horizontal reference plane H. This intersection also defines the axis A of the compression paddle 201. During use, both the horizontal reference plane H and the bottom reference plane P are disposed a predetermined distance above, and substantially parallel to, a breast compression platform. Vertical reference plane V is substantially orthogonal to the vertical reference plane V, the horizontal reference plane H, the breast platform, and each of the depicted cross sections. Further, the horizontal reference plane H is also orthogonal to each of the depicted cross sections. Each cross section is substantially parallel to the front reference plane R.

FIG. 4A depicts a cross section of the compression paddle 201 at a location proximate the front wall, more particularly, where the front wall 314 transitions to the top surface 218 and bottom wall 204. This cross section is defined by the top wall 218, bottom wall 204, and rounded side walls 208. The cross section defines a first shape that is substantially curved or bean-shaped. The central portion 212 is disposed a distance d above the bottom reference plane P. Each cross section is substantially parallel to the front reference plane R.

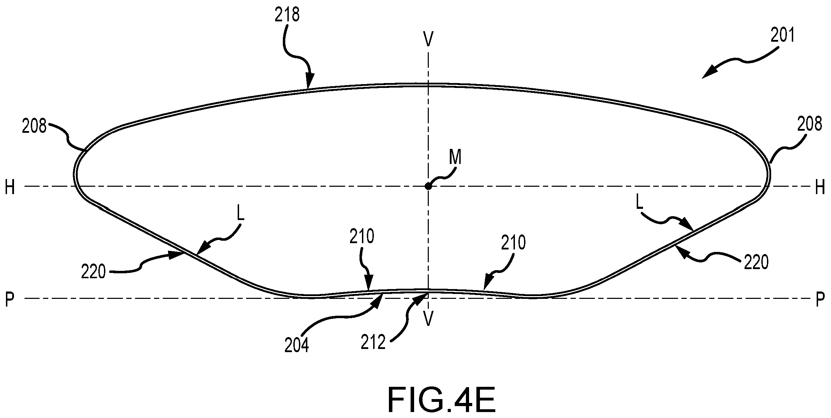

With each successive cross section depicted in FIGS. 4B-4E, the cross-sectional shape of the compression paddle 201 changes. As the cross section approaches the bracket end 216 of the compression paddle 201, the changes become more marked. For example, as can be seen, the distance d decreases as the distance away from the front wall 214 (as depicted in FIGS. 4B-4E) increases. Further, the curved shape originally depicted in the cross section of FIG. 4A is altered. Notably, in FIGS. 4D and 4E, the rounded outer ends of this curved shape flattens, as the cross section defines the access corners 220. In the above description, the cross section of the compression paddle 201 is defined by the entire outer surface of the compression paddle 201. However, in other examples, the cross section may only be defined by a portion of the outer surface of the paddle 201. For example, the cross section may be defined by the horizontal reference plane H and the surfaces disposed below that horizontal reference plane H. One reason for such a definition is that the top surface 218 of the compression paddle 201 is largely unchanged at each cross section. Another reason for such a definition is that a top surface 218 is not necessarily always present on every compression paddle. Indeed, the depicted figures depict so-called "hollow" compression paddles that have a hollow interior closed by an outer surface, including the top surface 218. In other examples, the complex bottom shapes of the paddles depicted and described herein (including but not limited to the access surfaces 220) may be utilized on any type of compression paddle. For example, existing compression paddles that utilize an "open" top (i.e., a paddle that does not include a top surface) may also benefit from the complex bottom paddle shapes described herein.

Again with reference to FIGS. 4A-4E, the shape of the compression paddle 201 may be defined based on the outer contours or the cross section of the compression paddle 201 itself. The compression paddle 201 has a front wall that is adjacent to the chest wall of the patient when during imaging procedures. The horizontal reference plane H is disposed at a midpoint M of the front wall (both in horizontal and vertical directions). The bottom wall 204 is configured to be adjacent to the breast of the patient during imaging procedures. The compression paddle 201 may be defined by multiple cross sections. a front cross section (e.g., a cross section closest to the front wall of the compression paddle 201) may be different than a rear cross section. The rear cross section may be disposed a predetermined distance from the front wall, for example, at the end of the compression paddle 201 proximate the bracket.

One aspect of the cross sections that may define the differences therebetween is the position of the bottom wall 204 relative to the bottom reference plane P. Proximate the front wall, the cross section has a central portion 212 intersecting the vertical reference plane P that is disposed a first predetermined distance below the horizontal reference plane H. At the rear cross section, a portion of the bottom wall 204 intersecting the vertical reference plane P is disposed a second predetermined distance below the horizontal reference plane H. In an example where the bottom surface 204 is pitched, the first distance is less than the second distance.

Another aspect of the cross sections that may define the differences therebetween is the configuration of the outer edge portions 210. The two outer edge portions at least partially define the bottom wall 204 and, in the first cross section, are disposed a first distance from the vertical reference plane V. At the second cross section, the two outer edge portions 210 are disposed a second distance from the vertical reference plane V. In such an example, the first distance is greater than the second distance. As the cross sections change in shape from the first cross section to the second cross section and beyond, the outer edge portions 210 include a substantially linear portion (at the first cross section) and a substantially curved portion (at the second cross section). These correspond to the linear and curved portions of the outer edge portions depicted in FIG. 3H. Additionally, FIGS. 4A-4E depict additional differences between the cross sections that may be defined by the outer contours of the compression paddle 201 boundary that defines the cross section. For example, the first cross section includes outer contours defined by a first boundary which may have a first curvature C1 (as depicted in FIG. 4A), while the second cross section includes outer contours defined by a second boundary different than the first boundary. In FIG. 4E, this second boundary is defined by a line which may be substantially straight or have a slight curvature different than that first curvature C1.

The complex shape of the compression paddle 201 may be further defined by the shape of the bottom wall 204. For example, the bottom wall 204 may include a generally concave contact surface 206 that is adjacent to the breast of the patient during imaging procedures. An outer extent of the contact surface 206 may be defined at least in part by two outer edge portions 210. The position of the outer edge portions 210 may vary in each cross section, such as those depicted in FIGS. 4A-4E. As depicted, a distance between the vertical reference plane V and the two outer edge portions 210 decreases as a distance from the front wall 214 increases. Additionally, a distance of the contact surface 206 below the horizontal reference plane H increases as the distance from the front wall 214 increases. As depicted in FIGS. 4A-4E, the plurality of cross sections include outer contours that change in shape as the distance from the front wall 214 increases. For example, the outer contours change from a curved shape defined by curvature C1 (as depicted in FIG. 4A) to a substantially linear shape defined by line L (as depicted in FIG. 4E) as the from the front wall 214 increases. Due to the configuration of the outer edge portions 210, a width of the contact surface 206 changes (e.g., narrows or decreases) as the distance from the front wall 214 increases.

Certain features of the hollow compression paddle depicted in FIGS. 3-4E may also be incorporated into paddles that lack the top surface of the above compression paddle. Such compression paddles, for example, are depicted in FIGS. 10A-12E and 14A-15C of U.S. Patent No. 9,782,135. Such compression paddles generally consist of a single-walled, injection molded element, a bottom surface of which compresses the breast. The sides of that single-walled element are bent or folded upwards so as to form a bowl-like shape, having a front wall connecting two lateral walls. A rear wall of the bowl-like shape is proximate a bracket with which the compression paddle is connected to a compression system of an x-ray imaging system. Thus, the single-walled element has a bottom compression surface; the top surface of that element generally forms the bottom of a bowl-like structure of the compression paddle. Such paddles may have a flat, concave, or convex surface across a whole or a part of the bowl-like structure.

The technologies disclosed herein may improve the usability of these bowl-like compression paddles, e.g., by raising the bracket portion thereof relative to the compression portion to give a technologist improved access to a breast during positioning and compression. This improved access is the result of the compression paddle including a bottom wall having a patient contact surface and a patient access surface. During a compression of the breast, the patient contact surface is in contact with the breast. The patient access surface, however, is disposed a distance above the patient contact surface so as to not contact the breast and provide access to the breast by a technologist. A transition wall having a generally smooth curvature is between and connects the patient contact surface and the patient access surface. In addition to eliminating sharp contact points, the generally smooth curvature of the transition wall may also reduce artifacts that may be formed by the compression paddle during imaging. A front wall of the compression paddle is configured to be in contact with a chest wall during compression. An interface wall is between and connects the patient contact surface and the front wall. Like the transition wall, the interface wall is a generally smooth curvature to reduce or eliminate pressure or pinch points, as well as artifacts.

Although x-ray translucent materials are utilized for the compression paddles described herein, image artifacts may still be formed under certain circumstances. Vertical surfaces may generate artifacts in an image because of the increased amount of material through which the x-ray radiation must pass. As such, in the following examples, vertical surfaces such as the front wall and lateral side walls are disposed outside of an imaging area to prevent generation of such artifacts. Sharp transitions from one surface to another (e.g., corners or intersecting edges) may also form image artifacts for similar reasons. As such, the compression paddles described below utilize smooth contours on all surfaces in the imaging area so as to reduce or prevent such artifacts.

The compression paddles described below incorporate additional features so as to improve manufacturability and conformance with the compression paddle with the breast. These include the use of ribs or other structures below a bracket portion of a compression paddle to increase rigidity thereof. The ribs also allow the compression paddle to be formed in a single injection molding process. The use of the smooth contours described above also reduces stress points in the paddle, while allowing the compression portion of the paddle to better conform to the shape of the breast. The compression portion of the paddle also lacks the above-described ribs, which allows the compression portion to deflect during breast compression. These smooth shapes and deflection capability helps reduce discomfort commonly associated with flat paddles.

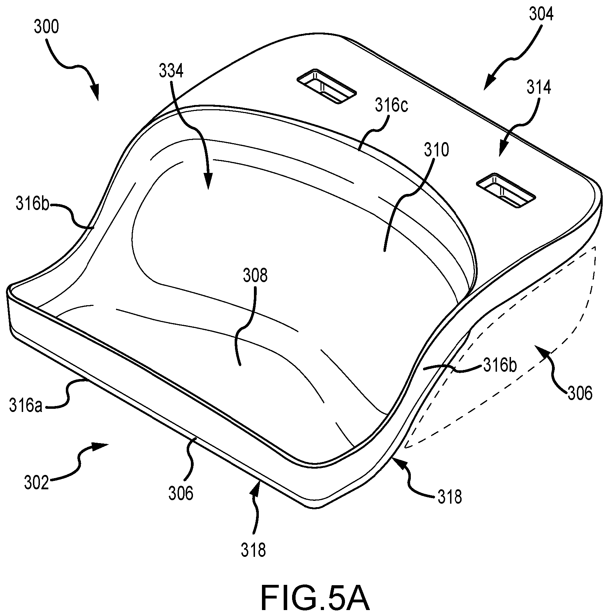

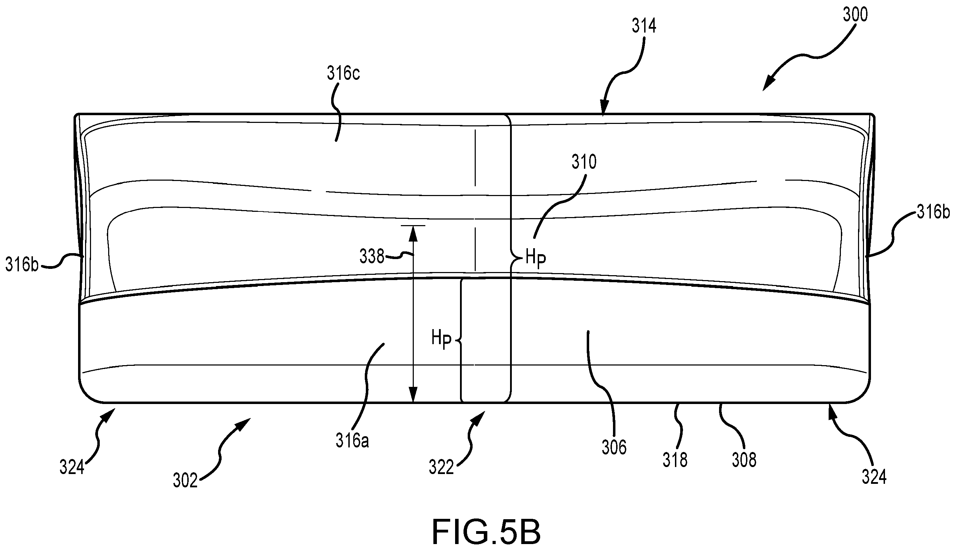

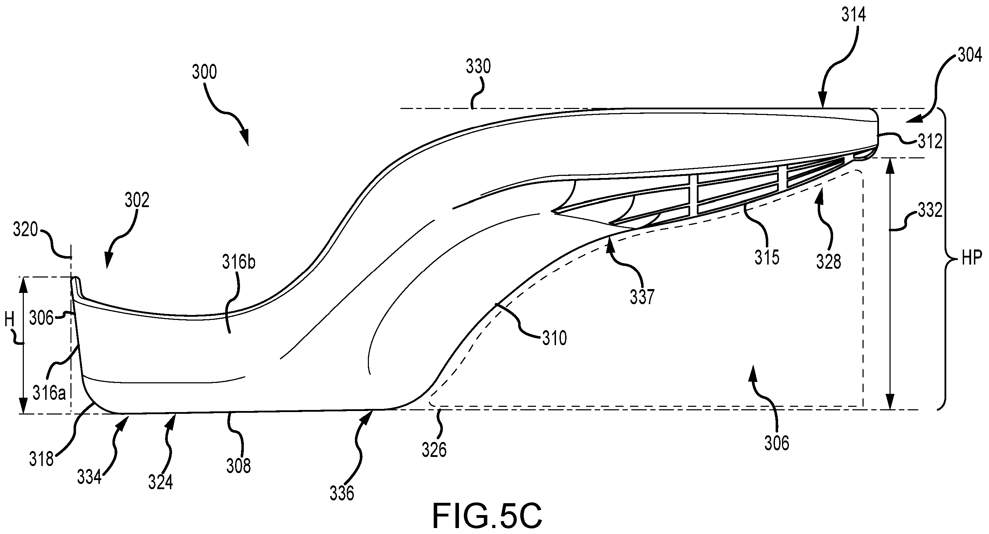

FIGS. 5A-5C are perspective, front, and side views, respectively, of an example of a bowl-like compression paddle 300, and are described concurrently. The compression paddle includes a compression portion 302 and a bracket portion 304. The compression portion 302 is configured to be disposed proximate the chest wall of a patient during compression, while the bracket portion 304 is used to secure the compression paddle 300 to a compression arm of an imaging system. The bracket portion 304 is raised relative to the compression portion 302 so as to define an access area 306 below at least a portion of the compression paddle 300, which allows a technologist to better access a patient breast during positioning. The access area 306 is depicted generally by dashed lines in FIGS. 5A and 5C. Of course, the compression paddle 300 is a three-dimensional object, as such, the so called "access area" is actually a volume primarily disclosed below the bracket portion 304 and an angled wall (in this case, transition wall 310) of the compression paddle 300. A front wall 306 is disposed so as to contact the chest wall of a patient. A bottom wall 308 acts as a compression surface for the breast during imaging procedures. The transition wall 310 extends towards the bracket portion 304 and at least partially defines the access area 306. A rear bracket wall 312 extends down from a surface 314 that defines the top-most extent of both the bracket portion 304 and the compression paddle 300 as a whole. A plurality of ribs 315 extend from the rear bracket wall 312 so as to provide structural rigidity to the compression paddle 300; more specifically, the ribs 315 provide structural rigidity to the bracket portion 304. The configuration of the compression portion 302, that is, of an open, bowl-like structure, allows for flexure of the compression portion 302 during compression of the breast. A boundary wall 316 generally defines the open, bowl-like structure. The boundary wall 316 includes a front boundary wall 316a, lateral boundary walls 316b, and a rear boundary wall 316c. The front boundary wall 316a is generally coextensive with the front wall 306.

As noted above, the front wall 306 is configured to be adjacent to and face a chest wall of a patient during imaging. The front wall 306 includes a front wall height H, a curved lower interface 318 that connects to the bottom wall 308, and a front reference plane 320. The bottom wall 308 extends away from the chest wall and, during imaging, is adjacent a length of a top of a compressed breast. The bottom wall 308 includes a generally central portion 322 and two outer edge portions 324 that at least partially define the central portion 322. The two outer edge portions 324 extend away from the front reference surface 320, for example, as depicted most clearly in the side view of FIG. 5C. The generally central portion 322 extends away from the front reference surface 320 a greater distance than the two outer edge portions 324. As such, the shape of the bottom wall 308 general corresponds to the outer shape of the compressed breast. The two outer edge portions 324 (or at least three points disposed thereon) define a bottom reference plane 326 that is substantially orthogonal to the front reference plane 320. The bottom reference plane 326, in this case, is generally a horizontal plane that is substantially consistent with the bottom surface of the paddle 300. The bracket portion 304 is distal from the front wall 306, and includes the paddle top surface 314 and a bracket underside surface 328. The paddle top surface 314 defines a top reference plane 330 that is substantially parallel to the bottom reference plane 326. The top reference plane 330 is disposed a maximum paddle height H_{P} above the bottom reference plane 326. This maximum paddle height H_{P} is greater than the front wall height H, which again helps improve technologist access to the breast.

The rear bracket wall 312 is disposed opposite the front wall 306 and connects the paddle top surface 314 and the bracket underside surface 328, and wherein the rear bracket wall terminates at a distance 332 above the bottom reference plane 326 that is greater than the front wall height H. In examples, such as that depicted in FIGS. 5A-5C, the bracket underside surface 328 is defined as a portion of the paddle 300 that is disposed directly below the flat paddle top surface 314 that defines the top reference plane 326. In examples, the lowermost portion of each of the plurality of ribs 315 (again, below the flat paddle top surface 314) act as the bracket underside surface 328. The angled transition wall 310 connects the bracket underside surface 328 and the bottom wall 308. Thus, the bracket portion 304 of the compression paddle 300 is considerably higher than the compression portion 302, allowing a technologist to have improved access to the breast during positioning and compression.

When the compression paddle 300 is viewed from above, for example, from the perspective of FIG. 5A, the bottom wall 308 and the transition wall 310 are substantially surrounded by the boundary or perimeter wall 316 that extends upward towards the top reference plane 330. Thus, viewed from this perspective, the bottom wall 308, the transition wall 310, and the boundary or perimeter wall 316 define a semi-bounded volume 334. In the depicted example, the bottom wall 308 and the transition wall 310 form a lower surface of the semi-bounded volume 334 where the bottom wall 308 is non-concave. More specifically, the bottom wall 308 is substantially flat. The transition wall 310 is substantially convex when viewed from above. The two lateral boundary walls 316b extend from the front boundary wall 316a to the rear boundary wall 316c, and slope generally upward along the transition wall 310. The bottom wall 308 is connected to each of the boundary walls 316 at a curved interface 318. As described above, an uppermost portion of the front boundary wall 316a has a front boundary wall height H above the bottom reference plane 326. An uppermost portion of the rear boundary wall 316c is connected to and approximately the same height as that of the top surface 314 of the bracket 304 portion. Thus, this rear boundary wall height above the bottom reference plane 326, is generally the same as the maximum paddle height H_{P}, such that the front boundary wall height H is less than the rear boundary wall height above the bottom reference plane 326. The two outer edge portions 324 of the bottom wall 308 are disposed proximate the two lateral boundary walls 316b. The bottom wall 308 also includes a proximate portion 334 that is disposed proximate the front boundary wall 316a and a distal portion 336 disposed distal from the front boundary wall 316a which in this example is generally level with the proximate portion 336. As depicted in FIG. 5C, a high portion 337 of the transition wall 310 is disposed a high portion distance 338 above the bottom reference plane 326. In the compression paddle 300 of FIG. 5A-5C, the high portion distance 338 is greater than the front boundary wall height H. As can be seem most clearly in FIG. 5A, the rear boundary wall 316c is generally curved along its length and is closest to the rear paddle wall 312 proximate a mid-point of that curvature. The ribs 315 can be seen as extending from the rear boundary wall 316c to the rear paddle wall 312.

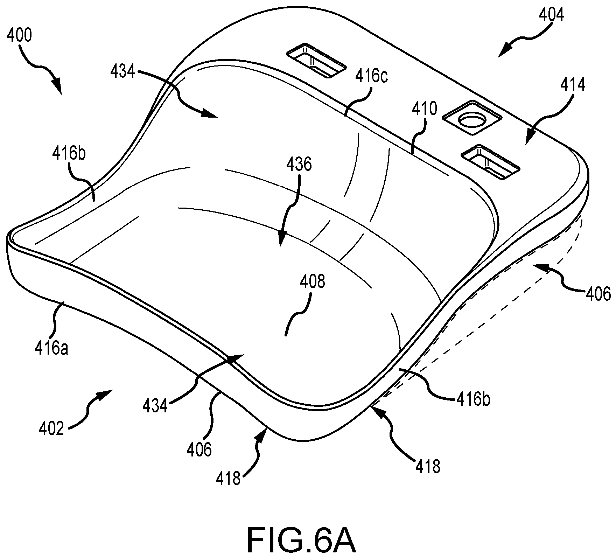

FIGS. 6A-6C are perspective, front, and side views, respectively, of another example of a compression paddle 400, and are described concurrently. The compression paddle includes a compression portion 402 and a bracket portion 404. The compression portion 402 is configured to be disposed proximate the chest wall of a patient during compression, while the bracket portion 404 is used to secure the compression paddle 400 to a compression arm of an imaging system. The bracket portion 404 is raised relative to the compression portion 402 so as to define an access area 406 below at least a portion of the compression paddle 400, which allows a technologist to better access a patient breast during positioning, as described above. A front wall 406 is disposed so as to contact the chest wall of a patient. A bottom wall 408 acts as a compression surface for the breast during imaging procedures. A transition wall 410 extends towards the bracket end 404 and at least partially defines the access area 406. A rear bracket wall 412 extends down from a surface 414 that defines the top-most extent of both the bracket portion 404 and the compression paddle 400. A plurality of ribs 415 extend from the rear bracket wall 412 so as to provide structural rigidity to the compression paddle 400, namely to the bracket portion 404. The open, bowl-like configuration of the compression end 402 allows for flexure of the compression portion 402 during compression of the breast. A boundary wall 416 generally defines the open, bowl-like structure. The boundary wall 416 includes a front boundary wall 416a, lateral boundary walls 416b, and a rear boundary wall 416c. The front boundary wall 416a is generally coextensive with the front wall 406.

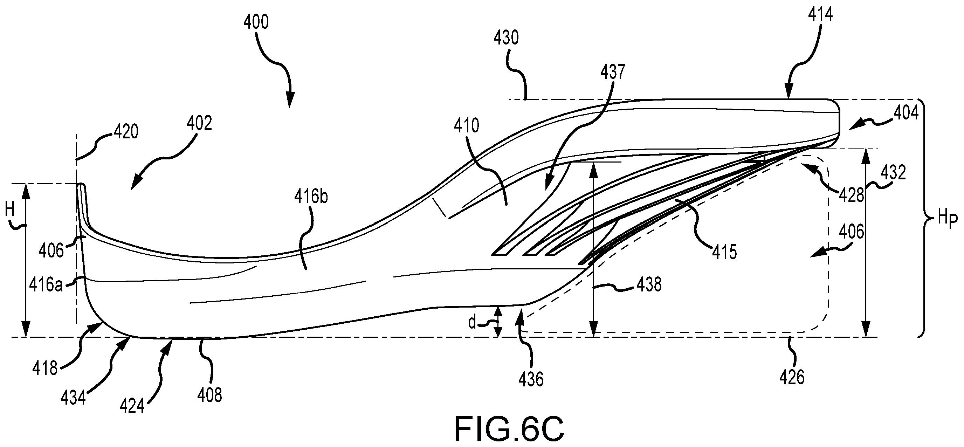

As noted above, the front wall 406 is configured to be adjacent to and face a chest wall of a patient during imaging. The front wall 406 includes a front wall height H, a curved lower interface 418 that connects to the bottom wall 408, and a front reference plane 420. The bottom wall 408 extends away from the chest wall and, during imaging, is adjacent a length of a top of a compressed breast. The bottom wall 408 includes a generally central portion 422 and two outer edge portions 424 that at least partially define the central portion 422. In this example, the generally central portion 422 is raised relative to the two outer edge portions 424, which extend away from the front reference surface 420, for example, as depicted most clearly in the side view of FIG. 6C. The two outer edge portions 424 (or at least three points disposed thereon) define the bottom reference plane 426 that is substantially orthogonal to the front reference plane 420. The bottom reference plane 426, in this case, is generally a horizontal plane that is defined by the lowermost points of the paddle 400. The bracket portion 404 is distal from the front wall 406, and includes the paddle top surface 414 and a bracket underside surface 428. The paddle top surface 414 defines a top reference plane 430 that is substantially parallel to the bottom reference plane 426. The top reference plane 430 is disposed a maximum paddle height H_{P} above the bottom reference plane 426. This maximum paddle height H_{P} is greater than the front wall height H, thus improving technologist access.

The rear bracket wall 412 is disposed opposite the front wall 406 and connects the paddle top surface 414 and the bracket underside surface 428, and wherein the rear bracket wall 412 terminates at a distance 432 above the bottom reference plane 426 that is greater than the front wall height H. In examples, such as that depicted in FIGS. 6A-6C, the bracket underside surface 428 is defined as a portion of the paddle 400 that is disposed directly below the flat paddle top surface 414 that defines the top reference plane 426. In examples, the lowermost portion of each of the plurality of ribs 415 (again, below the flat paddle top surface 414) act as the bracket underside surface 428. In this example, the ribs 415 extend to a location on the angled transition wall 410 that is lower than the front wall height H. The angled transition wall 410 connects the bracket underside surface 428 and the bottom wall 408. The bracket end 404 of the compression paddle 400 is considerably higher than the compression end 402, allowing a technologist to have improved access to the breast during positioning and compression.

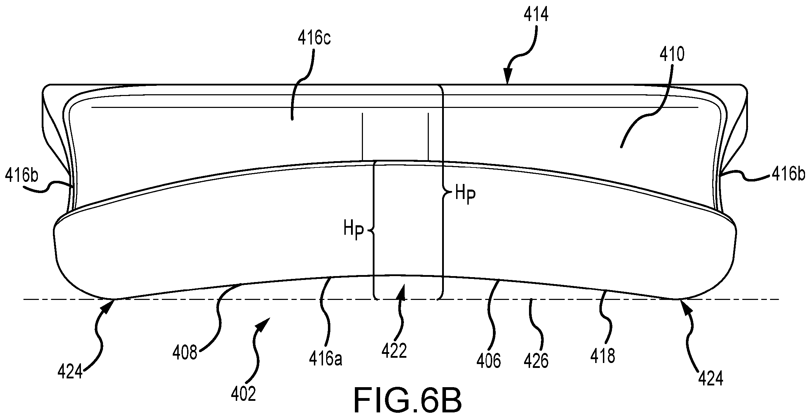

When the compression paddle 400 is viewed from above, for example, from the perspective of FIG. 6A, the bottom wall 408 and the transition wall 410 are substantially surrounded by the boundary or perimeter wall 416 that extends upward towards the top reference plane 430. Thus, viewed from this perspective, the bottom wall 408, the transition wall 410, and the boundary or perimeter wall 416 define a semi-bounded volume 434. In the depicted example, the bottom wall 408 and the transition wall 410 form a lower surface of the semi-bounded volume 434 where the bottom wall 408 is non-concave. More specifically, the bottom wall 408 is convex, as depicted in FIG. 6B. The transition wall 410 is substantially concave when viewed from above. The two lateral boundary walls 416b extend from the front boundary wall 416a to the rear boundary wall 416c, and slope generally upward along the transition wall 410. The bottom wall 408 is connected to each of the boundary walls 416 at a curved interface 418. As described above, an uppermost portion of the front boundary wall 416a has a front boundary wall height H above the bottom reference plane 426. An uppermost portion of the rear boundary wall 416c is connected to and approximately the same height as that of the top surface 414 of the bracket 404 portion. Thus, this rear boundary wall height above the bottom reference plane 426, is generally the same as the maximum paddle height H_{P}, such that the front boundary wall height H is less than the rear boundary wall height above the bottom reference plane 426. The two outer edge portions 424 of the bottom wall 408 are disposed proximate the two lateral boundary walls 416b. The bottom wall 408 also includes a proximate portion 434 that is disposed proximate the front boundary wall 416a and a distal portion 436 disposed distal from the front boundary wall 416a. The distal portion 436 is proximate the transition wall 410 and is disposed a distance d above the bottom reference plane 426. This distance d allows the compression portion 402 to flex upward without contacting a breast support platform disposed beneath. As depicted in FIG. 6C, a high portion 437 of the transition wall 410 is disposed a high portion distance 438 above the bottom reference plane 426. In the compression paddle 400 of FIG. 6A-6C, the high portion distance 438 is greater than the front boundary wall height H. As can be seem most clearly in FIG. 6A, the rear boundary wall 416c is generally parallel the rear paddle wall 412. The ribs 415 can be seen as extending from the rear boundary wall 416c to the rear paddle wall 412.

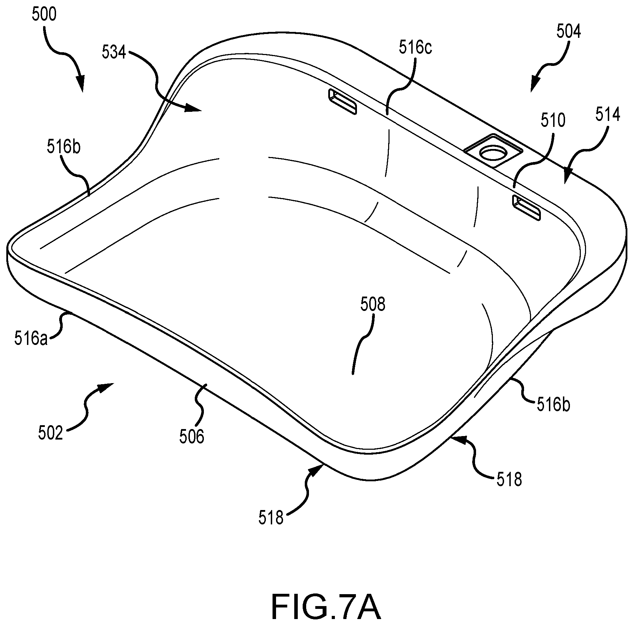

FIGS. 7A-7C are perspective, front, and side views, respectively, of another example of a compression paddle 500, and are described concurrently. The compression paddle includes a compression portion 502 and a bracket portion 504. The compression portion 502 is configured to be disposed proximate the chest wall of a patient during compression, while the bracket portion 504 is used to secure the compression paddle 500 to a compression arm of an imaging system. The bracket portion 504 is raised relative to the compression portion 502 so as to define an access area 506 below at least a portion of the compression paddle 500, which allows a technologist to better access a patient breast during positioning, as described above. A front wall 506 is disposed so as to contact the chest wall of a patient. A bottom wall 508 acts as a compression surface for the breast during imaging procedures. A transition wall 510 extends towards the bracket portion 504 and at least partially defines the access area 506. A rear bracket wall 512 extends down from a surface 514 that defines the top-most extent of both the bracket portion 504 and the compression paddle 500. A plurality of ribs 515 extend from the rear bracket wall 512 so as to provide structural rigidity to the compression paddle 500, namely to the bracket portion 504. The open, bowl-like configuration of the compression portion 502 allows for flexure of the compression portion 502 during compression of the breast. A boundary wall 516 generally defines the open, bowl-like structure. The boundary wall 516 includes a front boundary wall 516a, lateral boundary walls 516b, and a rear boundary wall 517C. The front boundary wall 516a is generally coextensive with the front wall 506.

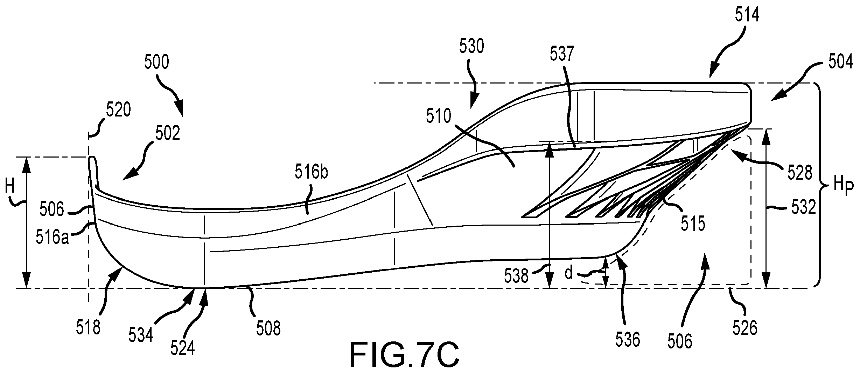

As noted above, the front wall 506 is configured to be adjacent to and face a chest wall of a patient during imaging. The front wall 506 includes a front wall height H, a curved lower interface 518 that connects to the bottom wall 508, and a front reference plane 520. The bottom wall 508 extends away from the chest wall and, during imaging, is adjacent a length of a top of a compressed breast. The bottom wall 508 includes a generally central portion 522 and two outer edge portions 524 that at least partially define the central portion 522. In this example, the generally central portion 522 is raised relative to the two outer edge portions 524, which extend away from the front reference surface 520, for example, as depicted most clearly in the side view of FIG. 7C. The two outer edge portions 524 (or at least three points disposed thereon) define the bottom reference plane 526 that is substantially orthogonal to the front reference plane 520. The bottom reference plane 526, in this case, is generally a horizontal plane that is defined by the lowermost points of the paddle 500. The bracket 504 is distal from the front wall 506, and includes the paddle top surface 514 and a bracket underside surface 528. The paddle top surface 514 defines a top reference plane 530 that is substantially parallel to the bottom reference plane 526. The top reference plane 530 is disposed a maximum paddle height H_{P} above the bottom reference plane 526. This maximum paddle height H_{P} is greater than the front wall height H, thereby improving technologist access.

The rear bracket wall 512 is disposed opposite the front wall 506 and connects the paddle top surface 514 and the bracket underside surface 528, and wherein the rear bracket wall 512 terminates at a distance 532 above the bottom reference plane 526 that is greater than the front wall height H. In examples, such as that depicted in FIGS. 7A-7C, the bracket underside surface 528 is defined as a portion of the paddle 500 that is disposed directly below the flat paddle top surface 514 that defines the top reference plane 526. In examples, the lowermost portion of each of the plurality of ribs 515 (again, below the flat paddle top surface 514) act as the bracket underside surface 528. In this example, the ribs 515 extend to a location on the angled transition wall 510 that is lower than the front wall height H. The angled transition wall 510 connects the bracket underside surface 528 and the bottom wall 508. The bracket end 504 of the compression paddle 500 is considerably higher than the compression end 502, allowing a technologist to have improved access to the breast during positioning and compression.

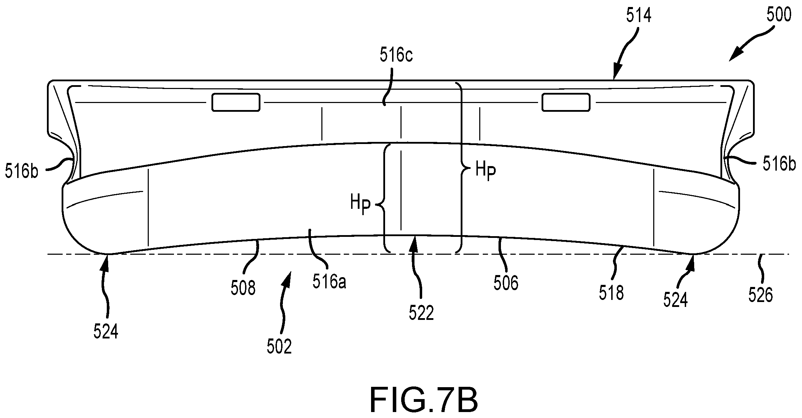

When the compression paddle 500 is viewed from above, for example, from the perspective of FIG. 7A, the bottom wall 508 and the transition wall 510 are substantially surrounded by the boundary or perimeter wall 516 that extends upward towards the top reference plane 530. Thus, viewed from this perspective, the bottom wall 508, the transition wall 510, and the boundary or perimeter wall 516 define a semi-bounded volume 534. In the depicted example, the bottom wall 508 and the transition wall 510 form a lower surface of the semi-bounded volume 534 where the bottom wall 508 is non-concave. More specifically, the bottom wall 508 is convex, as depicted in FIG. 7B. The transition wall 510 is substantially concave when viewed from above. The two lateral boundary walls 516b extend from the front boundary wall 516a to the rear boundary wall 517C, and slope generally upward along the transition wall 510. The bottom wall 508 is connected to each of the boundary walls 516 at a curved interface 518. As described above, an uppermost portion of the front boundary wall 516a has a front boundary wall height H above the bottom reference plane 526. An uppermost portion of the rear boundary wall 516C is connected to and approximately the same height as that of the top surface 514 of the bracket 504 portion. Thus, this rear boundary wall height above the bottom reference plane 526, is generally the same as the maximum paddle height H_{P}, such that the front boundary wall height H is less than the rear boundary wall height above the bottom reference plane 526. The two outer edge portions 524 of the bottom wall 508 are disposed proximate the two lateral boundary walls 516b. The bottom wall 508 also includes a proximate portion 534 that is disposed proximate the front boundary wall 516a and a distal portion 536 disposed distal from the front boundary wall 516a. The distal portion 536 is proximate transition wall 510 and is disposed a distance d above the bottom reference plate 526. This distance d allows the compression portion 502 to flex upwards without contacting a breast support platform disposed beneath. As depicted in FIG. 7C, a high portion 537 of the transition wall 510 is disposed a high portion distance 538 above the bottom reference plane 526. In the compression paddle 500 of FIG. 7A-7C, the high portion distance 538 is greater than the front boundary wall height H. As can be seem most clearly in FIG. 7A, the rear boundary wall 517C is generally parallel the rear paddle wall 512. The ribs 515 can be seen as extending from the rear boundary wall 516C to the rear paddle wall 512.

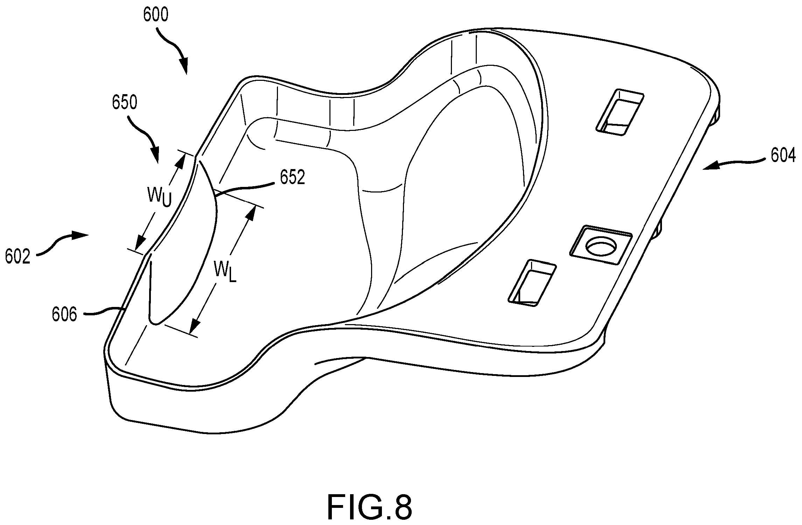

FIG. 8 is a rear perspective view of another example of a compression paddle 600. The compression paddle 600 includes a compression end 602 and a bracket end 604 and is generally configured consistent with the compression paddle 300 of FIGS. 5A-5C. However, the compression paddles 400, 500 depicted in FIGS. 6A-7C may be similarly modified to include such a recess. In this example, the front wall 606 at least partially defines a recess or indentation 650. The recess 650 is defined by an upper width Wu and a lower width W_{L} that is wider than the upper width Wu. The recess 650 includes a curved wall 652 that is further disposed at an angle to that of the front wall 606. This recess 650 is particularly advantageous for obtaining unobstructed views of breasts that include silicone or saline breast implants. The curved wall 652 of the recess 650 is focused to the focal source of the imaging system. The curved wall 652 of the recess 650 pushes the implant out of the compression area below the paddle 600. In examples, the upper width Wu may be about 50 percent of the lower width W_{L}. In other examples, the upper width W_{L} may be between about 45-60 percent, between about 40-70 percent, or between about 35-80 percent of the lower width W_{L}

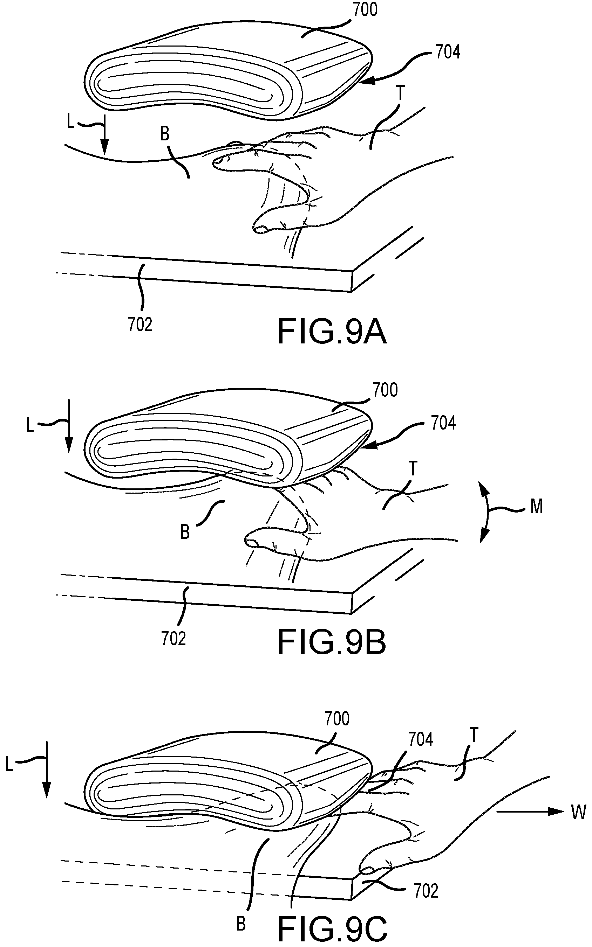

FIGS. 9A-9C depict a method of using a compression paddle 700 prior to an imaging procedure. In the depicted figures, the torso of the patient is not depicted, for clarity. Additionally, a compression paddle 700 similar to that depicted in FIGS. 3-4E is depicted. The other paddles depicted above in FIGS. 5A-8 may also be utilized with similar results, due to the access areas located below the paddle brackets, as described above. In FIGS. 9A-9C, an approximate location of the breast B and technician T manipulating the breast B on a platform 702 is depicted. Once the breast is placed on the platform 702, it is held in place by the technician T as the compression paddle 700 is lowered L, as depicted in FIG. 9A. FIG. 9B depicts the condition where the compression paddle 700 first contacts the breast B. The technician T may move M her hand so as to position the breast B as required. With prior art compression paddles, this position, with the compression paddle in initial contact with the breast, may begin to cause interference between the technician and the compression paddle. The access corners 704 of the present compression paddle 700, however, allow the technician T to move her hand relative to the breast B, with minimal, if any, interference with the compression paddle 700. In FIG. 9C, the compression paddle 700 is further lowered L, leading to further compression. Due to the configuration of the compression paddle 700, namely the access corners 704, the technician T may manipulate and position the breast B for longer without fear of her hand being caught between the compression paddle 700 and the breast B. Thus, the technician T is able to maintain contact with the breast B for longer, prior to withdrawing W her hand and beginning imaging procedures.

## Claims (Claims for the following Contracting State(s): AT, BE, CH, DK, ES, FR, GB, GR, IT, LI, LU, NL, SE, MC, PT, IE, SI, LT, LV, FI, RO, MK, CY, AL, TR, BG, CZ, EE, HU, PL, SK, HR, IS, MT, NO, RS, SM)

1. A breast compression paddle (300) for an imaging system, the breast compression paddle (300) comprising:
a plurality of boundary walls comprising a front boundary wall (316a), a rear boundary wall (316c), and two lateral boundary walls (316b) extending from the front boundary wall (316a) to the rear boundary wall (316c);
a bracket portion (304) extending from the rear boundary wall (316c) away from the front boundary wall (316a); and
a bottom wall (308) and a transition wall (310) connected to each of the boundary walls at a curved interface (318), wherein the bottom wall (308) comprises at least three points defining a bottom reference plane (326), and wherein an uppermost portion of the front boundary wall (316a) is disposed at a front boundary wall (316a) height above the bottom reference plane (326), and wherein an uppermost portion of the rear boundary wall (316c) is disposed at a rear boundary wall (316c) height above the bottom reference plane (326), and wherein the front boundary wall (316a) height is less than the rear boundary wall (316c) height, **characterized in that**
the bracket portion (304) is raised relative to a compression portion of the paddle (300) so as to define an access area (306) below at least a portion of the compression paddle (300), and
wherein the transition wall (310) extends from the bottom wall (308) towards the bracket portion (304) and at least partially defines the access area (306).

2. The breast compression paddle (300) of claim 1, wherein the bottom wall (308) comprises a raised central portion and a plurality of outer edge portions, and wherein the at least three points are disposed on the plurality of outer edge portions.

3. The breast compression paddle (300) of claim 1, wherein the bottom wall (308) comprises two outer edge portions disposed proximate the two lateral boundary walls (316b), and a central portion substantially level with the two outer edge portions.

4. The breast compression paddle (300) of claim 1, wherein the bottom wall (308) comprises a proximate portion disposed proximate to the front boundary wall (316a) and a distal portion disposed distal from the front boundary wall (316a) and wherein the distal portion is disposed a distal portion distance above the bottom reference plane (326).

5. The breast compression paddle (300) of any of claims 1-4, wherein the bracket portion (304) is connected to the rear boundary wall (316c) and comprises a rear paddle wall disposed opposite from the rear boundary wall (316c) and disposed substantially parallel to the rear boundary wall (316c).

6. The breast compression paddle (300) of claim 5, further comprising a plurality of ribs spanning from the rear boundary wall (316c) to the rear paddle wall.

7. The breast compression paddle (300) of claim 6, wherein at least one of the plurality of ribs contacts at least a portion of the bottom wall (308).

8. The breast compression paddle (300) of any of claims 1-7, wherein at least a portion of the transition wall (310) is convex.

9. The breast compression paddle (300) of any of claims 1-8, wherein the front boundary wall (316a) defines a recess having an upper width disposed proximate the uppermost portion of the front boundary wall (316a) and a lower width disposed proximate the bottom wall (308), wherein the lower width is greater than the upper width.

10. The breast compression paddle (300) of claim 9, wherein the recess is disposed at an angle to the front boundary wall (316a).

11. The breast compression paddle (300) of any of claims 1-10, utilizing smooth contours on all surfaces in the imaging area so as to reduce or prevent such artifacts.

12. The compression paddle (300) of any of claims 1, further comprising ribs that extend to a location on the transition wall (310) that is lower than the front boundary wall (316a) height.

13. The compression paddle (300) of any claims 1-12, comprising an open, bowl-like configuration.

14. The compression paddle (300) of any of the claims 1-13, wherein the transition wall (310) wall has a generally smooth curvature that connects the bottom wall (308) and a bottom surface of the bracket portion (304).

## Claims (Claims for the following Contracting State(s): DE)

1. A breast compression paddle (300) for an imaging system, the breast compression paddle (300) comprising:
a plurality of boundary walls comprising a front boundary wall (316a), a rear boundary wall (316c), and two lateral boundary walls (316b) extending from the front boundary wall (316a) to the rear boundary wall (316c);
a bracket portion (304) extending from the rear boundary wall (316c) away from the front boundary wall (316a); and
a bottom wall (308) and a transition wall (310) connected to each of the boundary walls at a curved interface (318), wherein the bottom wall (308) comprises at least three points defining a bottom reference plane (326), and wherein an uppermost portion of the front boundary wall (316a) is disposed at a front boundary wall (316a) height above the bottom reference plane (326), and wherein an uppermost portion of the rear boundary wall (316c) is disposed at a rear boundary wall (316c) height above the bottom reference plane (326), and wherein the front boundary wall (316a) height is less than the rear boundary wall (316c) height, **characterized in that**
the bracket portion (304) is raised relative to a compression portion of the paddle (300) so as to define an access area (306) below at least a portion of the compression paddle (300), and
wherein the transition wall (310) extends from the bottom wall (308) towards the bracket portion (304) and at least partially defines the access area (306), and
the transition wall (310) is substantially convex when viewed from above.

2. The breast compression paddle (300) of claim 1, wherein the bottom wall (308) comprises a raised central portion and a plurality of outer edge portions, and wherein the at least three points are disposed on the plurality of outer edge portions.

3. The breast compression paddle (300) of claim 1, wherein the bottom wall (308) comprises two outer edge portions disposed proximate the two lateral boundary walls (316b), and a central portion substantially level with the two outer edge portions.

4. The breast compression paddle (300) of claim 1, wherein the bottom wall (308) comprises a proximate portion disposed proximate to the front boundary wall (316a) and a distal portion disposed distal from the front boundary wall (316a) and wherein the distal portion is disposed a distal portion distance above the bottom reference plane (326).

5. The breast compression paddle (300) of any of claims 1-4, wherein the bracket portion (304) is connected to the rear boundary wall (316c) and comprises a rear paddle wall disposed opposite from the rear boundary wall (316c) and disposed substantially parallel to the rear boundary wall (316c).

6. The breast compression paddle (300) of claim 5, further comprising a plurality of ribs spanning from the rear boundary wall (316c) to the rear paddle wall.

7. The breast compression paddle (300) of claim 6, wherein at least one of the plurality of ribs contacts at least a portion of the bottom wall (308).

8. The breast compression paddle (300) of any of claims 1-7, wherein at least a portion of the transition wall (310) is convex.

9. The breast compression paddle (300) of any of claims 1-8, wherein the front boundary wall (316a) defines a recess having an upper width disposed proximate the uppermost portion of the front boundary wall (316a) and a lower width disposed proximate the bottom wall (308), wherein the lower width is greater than the upper width.

10. The breast compression paddle (300) of claim 9, wherein the recess is disposed at an angle to the front boundary wall (316a).

11. The breast compression paddle (300) of any of claims 1-10, utilizing smooth contours on all surfaces in the imaging area so as to reduce or prevent such artifacts.

12. The compression paddle (300) of any of claims 1, further comprising ribs that extend to a location on the transition wall (310) that is lower than the front boundary wall (316a) height.

13. The compression paddle (300) of any claims 1-12, comprising an open, bowl-like configuration.

14. The compression paddle (300) of any of the claims 1-13, wherein the transition wall (310) wall has a generally smooth curvature that connects the bottom wall (308) and a bottom surface of the bracket portion (304).

## Patentansprüche (Patentansprüche für folgende(n) Vertragsstaat(en): AT, BE, CH, DK, ES, FR, GB, GR, IT, LI, LU, NL, SE, MC, PT, IE, SI, LT, LV, FI, RO, MK, CY, AL, TR, BG, CZ, EE, HU, PL, SK, HR, IS, MT, NO, RS, SM)

1. Brustkompressionspaddel (300) für ein Bildgebungssystem, wobei das Brustkompressionspaddel (300) umfasst:
mehrere Begrenzungswände, die eine vordere Begrenzungswand (316a), eine hintere Begrenzungswand (316c) und zwei seitliche Begrenzungswände (316b) umfassen, die von der vorderen Begrenzungswand (316a) zu der hinteren Begrenzungswand (316c) verlaufen;
einen Halterungsabschnitt (304), der sich von der hinteren Begrenzungswand (316c) weg von der vorderen Begrenzungswand (316a) erstreckt; und
eine Bodenwand (308) und eine Übergangswand (310), die mit jeder der Begrenzungswände an einer gekrümmten Schnittstelle (318) verbunden sind, wobei die Bodenwand (308) wenigstens drei Punkte umfasst, die eine untere Referenzebene (326) definieren, und wobei ein oberster Abschnitt der vorderen Begrenzungswand (316a) in einer Höhe der vorderen Begrenzungswand (316a) über der unteren Referenzebene (326) angeordnet ist, und wobei ein oberster Abschnitt der hinteren Begrenzungswand (316c) in einer Höhe der hinteren Begrenzungswand (316c) über der unteren Referenzebene (326) angeordnet ist und wobei die Höhe der vorderen Begrenzungswand (316a) kleiner als die Höhe der hinteren Begrenzungswand (316c) ist, **dadurch gekennzeichnet, dass**
der Halterungsabschnitt (304) relativ zu einem Kompressionsabschnitt des Paddels (300) erhöht ist, um einen Zugangsbereich (306) unter wenigstens einem Abschnitt des Kompressionspaddels (300) zu definieren, und
wobei sich die Übergangswand (310) von der Bodenwand (308) zu dem Halterungsabschnitt (304) erstreckt und den Zugangsbereich (306) wenigstens teilweise definiert.

2. Brustkompressionspaddel (300) nach Anspruch 1, wobei die Bodenwand (308) einen erhöhten Mittelabschnitt und mehrere Außenkantenabschnitte umfasst und wobei die wenigstens drei Punkte an den mehreren Außenkantenabschnitten angeordnet sind.

3. Brustkompressionspaddel (300) nach Anspruch 1, wobei die Bodenwand (308) zwei äußere Randabschnitte, die nahe der beiden seitlichen Begrenzungswände (316b) angeordnet sind, und einen mittleren Abschnitt, der im Wesentlichen auf Höhe der beiden äußeren Randabschnitte ist, umfasst.

4. Brustkompressionspaddel (300) nach Anspruch 1, wobei die Bodenwand (308) einen proximalen Abschnitt, der nahe der vorderen Begrenzungswand (316a) angeordnet ist, und einen distalen Abschnitt, der distal von der vorderen Begrenzungswand (316a) angeordnet ist, umfasst und wobei der distale Abschnitt in einem distalen Abschnitt-Abstand über der unteren Referenzebene (326) angeordnet ist.

5. Brustkompressionspaddel (300) nach einem der Ansprüche 1-4, wobei der Halterungsabschnitt (304) mit der hinteren Begrenzungswand (316c) verbunden ist und eine hintere Paddelwand umfasst, die gegenüber der hinteren Begrenzungswand (316c) angeordnet und im Wesentlichen parallel zu der hinteren Begrenzungswand (316c) angeordnet ist.

6. Brustkompressionspaddel (300) nach Anspruch 5, ferner umfassend mehrere Rippen, die von der hinteren Begrenzungswand (316c) zu der hinteren Paddelwand verlaufen.

7. Brustkompressionspaddel (300) nach Anspruch 6, wobei wenigstens eine der mehreren Rippen wenigstens einen Abschnitt der Bodenwand (308) berührt.

8. Brustkompressionspaddel (300) nach einem der Ansprüche 1-7, wobei wenigstens ein Abschnitt der Übergangswand (310) konvex ist.

9. Brustkompressionspaddel (300) nach einem der Ansprüche 1-8, wobei die vordere Begrenzungswand (316a) eine Aussparung definiert, die eine obere Breite aufweist, die nahe dem obersten Abschnitt der vorderen Begrenzungswand (316a) angeordnet ist, und eine untere Breite, die nahe der unteren Wand (308) angeordnet ist, wobei die untere Breite größer als die obere Breite ist.

10. Brustkompressionspaddel (300) nach Anspruch 9, wobei die Vertiefung in einem Winkel zu der vorderen Begrenzungswand (316a) angeordnet ist.

11. Brustkompressionspaddel (300) nach einem der Ansprüche 1-10, das glatte Konturen an allen Oberflächen in dem Bildgebungsbereich verwendet, um solche Artefakte zu verringern oder zu verhindern.

12. Kompressionspaddel (300) nach einem der Ansprüche 1, ferner umfassend Rippen, die zu einer Stelle an der Übergangswand (310) verlaufen, die niedriger als die Höhe der vorderen Begrenzungswand (316a) ist.

13. Kompressionspaddel (300) nach einem der Ansprüche 1-12, umfassend eine offene, schalenartige Konfiguration.

14. Kompressionspaddel (300) nach einem der Ansprüche 1-13, wobei die Wand der Übergangswand (310) eine allgemein glatte Krümmung aufweist, die die Bodenwand (308) und eine Bodenfläche des Halterungsabschnitts (304) verbindet.

## Patentansprüche (Patentansprüche für folgende(n) Vertragsstaat(en): DE)

1. Brustkompressionspaddel (300) für ein Bildgebungssystem, wobei das Brustkompressionspaddel (300) umfasst:
mehrere Begrenzungswände, die eine vordere Begrenzungswand (316a), eine hintere Begrenzungswand (316c) und zwei seitliche Begrenzungswände (316b), die von der vorderen Begrenzungswand (316a) zu der hinteren Begrenzungswand (316c) verlaufen, umfassen;
einen Halterungsabschnitt (304), der sich von der hinteren Begrenzungswand (316c) weg von der vorderen Begrenzungswand (316a) erstreckt; und
eine Bodenwand (308) und eine Übergangswand (310), die mit jeder der Begrenzungswände an einer gekrümmten Schnittstelle (318) verbunden sind, wobei die Bodenwand (308) wenigstens drei Punkte umfasst, die eine untere Referenzebene (326) definieren, und wobei ein oberster Abschnitt der vorderen Begrenzungswand (316a) in einer Höhe der vorderen Begrenzungswand (316a) über der unteren Referenzebene (326) angeordnet ist, und wobei ein oberster Abschnitt der hinteren Begrenzungswand (316c) in einer Höhe der hinteren Begrenzungswand (316c) über der unteren Referenzebene (326) angeordnet ist und wobei die Höhe der vorderen Begrenzungswand (316a) kleiner als die Höhe der hinteren Begrenzungswand (316c) ist, **dadurch gekennzeichnet, dass**
der Halterungsabschnitt (304) relativ zu einem Kompressionsabschnitt des Paddels (300) erhöht ist, um einen Zugangsbereich (306) unter wenigstens einem Abschnitt des Kompressionspaddels (300) zu definieren, und
wobei sich die Übergangswand (310) von der Bodenwand (308) in Richtung zu dem Halterungsabschnitt (304) erstreckt und den Zugangsbereich (306) wenigstens teilweise definiert, und
die Übergangswand (310) in Draufsicht im Wesentlichen konvex ist.

2. Brustkompressionspaddel (300) nach Anspruch 1, wobei die Bodenwand (308) einen erhöhten Mittelabschnitt und mehrere Außenkantenabschnitte umfasst und wobei die wenigstens drei Punkte an den mehreren Außenkantenabschnitten angeordnet sind.

3. Brustkompressionspaddel (300) nach Anspruch 1, wobei die Bodenwand (308) zwei äußere Randabschnitte, die nahe der beiden seitlichen Begrenzungswänden (316b) angeordnet sind, und einen mittleren Abschnitt, der im Wesentlichen auf Höhe der beiden äußeren Randabschnitte ist, umfasst.

4. Brustkompressionspaddel (300) nach Anspruch 1, wobei die Bodenwand (308) einen proximalen Abschnitt, der nahe der vorderen Begrenzungswand (316a) angeordnet ist, und einen distalen Abschnitt, der distal von der vorderen Begrenzungswand (316a) angeordnet ist, umfasst und wobei der distale Abschnitt in einem distalen Abschnitt-Abstand über der unteren Referenzebene (326) angeordnet ist.

5. Brustkompressionspaddel (300) nach einem der Ansprüche 1-4, wobei der Halterungsabschnitt (304) mit der hinteren Begrenzungswand (316c) verbunden ist und eine hintere Paddelwand umfasst, die gegenüber der hinteren Begrenzungswand (316c) angeordnet und im Wesentlichen parallel zu der hinteren Begrenzungswand (316c) angeordnet ist.

6. Brustkompressionspaddel (300) nach Anspruch 5, ferner umfassend mehrere Rippen, die von der hinteren Begrenzungswand (316c) zu der hinteren Paddelwand verlaufen.

7. Brustkompressionspaddel (300) nach Anspruch 6, wobei wenigstens eine der mehreren Rippen wenigstens einen Abschnitt der Bodenwand (308) berührt.

8. Brustkompressionspaddel (300) nach einem der Ansprüche 1-7, wobei wenigstens ein Abschnitt der Übergangswand (310) konvex ist.

9. Brustkompressionspaddel (300) nach einem der Ansprüche 1-8, wobei die vordere Begrenzungswand (316a) eine Aussparung definiert, die eine obere Breite aufweist, die nahe dem obersten Abschnitt der vorderen Begrenzungswand (316a) angeordnet ist, und eine untere Breite, die nahe der unteren Wand (308) angeordnet ist, wobei die untere Breite größer als die obere Breite ist.

10. Brustkompressionspaddel (300) nach Anspruch 9, wobei die Vertiefung in einem Winkel zu der vorderen Begrenzungswand (316a) angeordnet ist.

11. Brustkompressionspaddel (300) nach einem der Ansprüche 1-10, das glatte Konturen an allen Oberflächen in dem Bildgebungsbereich verwendet, um solche Artefakte zu verringern oder zu verhindern.

12. Kompressionspaddel (300) nach einem der Ansprüche 1, ferner umfassend Rippen, die zu einer Stelle an der Übergangswand (310) verlaufen, die niedriger als die Höhe der vorderen Begrenzungswand (316a) ist.

13. Kompressionspaddel (300) nach einem der Ansprüche 1-12, umfassend eine offene, schalenartige Konfiguration.

14. Kompressionspaddel (300) nach einem der Ansprüche 1-13, wobei die Wand der Übergangswand (310) eine allgemein glatte Krümmung aufweist, die die Bodenwand (308) und eine Bodenfläche des Halterungsabschnitts (304) verbindet.

## Revendications (Revendications pour l'(les) Etat(s) contractant(s) suivant(s): AT, BE, CH, DK, ES, FR, GB, GR, IT, LI, LU, NL, SE, MC, PT, IE, SI, LT, LV, FI, RO, MK, CY, AL, TR, BG, CZ, EE, HU, PL, SK, HR, IS, MT, NO, RS, SM)

1. Plaque de compression du sein (300) pour un système d'imagerie, la plaque de compression du sein (300) comprenant :
une pluralité de parois de délimitation comprenant une paroi de délimitation avant (316a), une paroi de délimitation arrière (316c) et deux parois de délimitation latérales (316b) s'étendant de la paroi de délimitation avant (316a) à la paroi de délimitation arrière (316c) ;
une partie de support (304) s'étendant depuis la paroi de délimitation arrière (316c) à l'écart de la paroi de délimitation avant (316a) ; et
une paroi inférieure (308) et une paroi de transition (310) reliées à chacune des parois de délimitation au niveau d'une interface courbe (318), la paroi inférieure (308) comprenant au moins trois points définissant un plan de référence inférieur (326), et une partie supérieure de la paroi de délimitation avant (316a) étant disposée à une hauteur de paroi de délimitation avant (316a) au-dessus du plan de référence inférieur (326), et une partie supérieure de la paroi de délimitation arrière (316c) étant disposée à une hauteur de paroi de délimitation arrière (316c) au-dessus du plan de référence inférieur (326), et la hauteur de paroi de délimitation avant (316a) étant inférieure à la hauteur de paroi de délimitation arrière (316c), **caractérisé en ce que**
la partie de support (304) est surélevée par rapport à une partie de compression de la plaque (300) de manière à définir une zone d'accès (306) sous au moins une partie de la plaque de compression (300), et
la paroi de transition (310) s'étendant depuis la paroi inférieure (308) vers la partie de support (304) et définissant au moins partiellement la zone d'accès (306).

2. Plaque de compression du sein (300) selon la revendication 1, la paroi inférieure (308) comprenant une partie centrale surélevée et une pluralité de parties de bord extérieures, et les au moins trois points étant disposés sur la pluralité de parties de bord extérieures.

3. Plaque de compression du sein (300) selon la revendication 1, la paroi inférieure (308) comprenant deux parties de bord extérieures disposées à proximité des deux parois de délimitation latérales (316b), et une partie centrale sensiblement au niveau des deux parties de bord extérieures.

4. Plaque de compression du sein (300) selon la revendication 1, la paroi inférieure (308) comprenant une partie proximale disposée à proximité de la paroi de délimitation avant (316a) et une partie distale disposée à distance de la paroi de délimitation avant (316a) et la partie distale étant disposée à une distance de partie distale au-dessus du plan de référence inférieur (326).

5. Plaque de compression du sein (300) selon l'une quelconque des revendications 1 à 4, la partie de support (304) étant reliée à la paroi de délimitation arrière (316c) et comprenant une paroi de plaque arrière disposée à l'opposé de la paroi de délimitation arrière (316c) et disposée sensiblement parallèlement à la paroi de délimitation arrière (316c).

6. Plaque de compression du sein (300) selon la revendication 5, comprenant en outre une pluralité de nervures s'étendant de la paroi de délimitation arrière (316c) à la paroi de plaque arrière.

7. Plaque de compression du sein (300) selon la revendication 6, au moins une de la pluralité de nervures étant en contact avec au moins une partie de la paroi inférieure (308).

8. Plaque de compression du sein (300) selon l'une quelconque des revendications 1 à 7, au moins une partie de la paroi de transition (310) étant convexe.

9. Plaque de compression du sein (300) selon l'une quelconque des revendications 1 à 8, la paroi de délimitation avant (316a) définissant un évidement ayant une largeur supérieure disposée à proximité de la partie supérieure de la paroi de délimitation avant (316a) et une largeur inférieure disposée à proximité de la paroi inférieure (308), la largeur inférieure étant plus grande que la largeur supérieure.

10. Plaque de compression du sein (300) selon la revendication 9, l'évidement étant disposé selon un angle par rapport à la paroi de délimitation avant (316a).

11. Plaque de compression du sein (300) selon l'une quelconque des revendications 1 à 10, utilisant des contours lisses sur toutes les surfaces dans la zone d'imagerie de manière à reduire ou à empêcher de tels artefacts.

12. Plaque de compression (300) selon l'une quelconque des revendications 1, comprenant en outre des nervures qui s'étendent jusqu'à un emplacement sur la paroi de transition (310) qui est inférieur à la hauteur de la paroi de délimitation avant (316a).

13. Plaque de compression (300) selon l'une quelconque des revendications 1 à 12, comprenant une configuration ouverte en forme de bol.

14. Plaque de compression (300) selon l'une quelconque des revendications 1 à 13, la paroi de transition (310) ayant une courbure généralement lisse qui relie la paroi inférieure (308) et une surface inférieure de la partie de support (304).

## Revendications (Revendications pour l'(les) Etat(s) contractant(s) suivant(s): DE)

1. Plaque de compression du sein (300) pour un système d'imagerie, la plaque de compression du sein (300) comprenant :
une pluralité de parois de délimitation comprenant une paroi de délimitation avant (316a), une paroi de délimitation arrière (316c) et deux parois de délimitation latérales (316b) s'étendant de la paroi de délimitation avant (316a) à la paroi de délimitation arrière (316c) ;
une partie de support (304) s'etendant depuis la paroi de délimitation arrière (316c) à l'écart de la paroi de délimitation avant (316a) ; et
une paroi inférieure (308) et une paroi de transition (310) reliées à chacune des parois de délimitation au niveau d'une interface courbe (318), la paroi inférieure (308) comprenant au moins trois points définissant un plan de référence inférieur (326), et une partie supérieure de la paroi de délimitation avant (316a) étant disposée à une hauteur de paroi de délimitation avant (316a) au-dessus du plan de référence inférieur (326), et une partie supérieure de la paroi de délimitation arrière (316c) étant disposée à une hauteur de paroi de délimitation arrière (316c) au-dessus du plan de référence inférieur (326), et la hauteur de paroi de délimitation avant (316a) étant inférieure à la hauteur de paroi de délimitation arrière (316c), **caractérisé en ce que**
la partie de support (304) est surélevée par rapport à une partie de compression de la plaque (300) de manière à définir une zone d'accès (306) sous au moins une partie de la plaque de compression (300), et
la paroi de transition (310) s'étendant depuis la paroi inférieure (308) vers la partie de support (304) et définissant au moins partiellement la zone d'accès (306), et
la paroi de transition (310) étant sensiblement convexe lorsque vue de dessus.

2. Plaque de compression du sein (300) selon la revendication 1, la paroi inférieure (308) comprenant une partie centrale surélevée et une pluralité de parties de bord extérieures, et les au moins trois points étant disposés sur la pluralité de parties de bord extérieures.

3. Plaque de compression du sein (300) selon la revendication 1, la paroi inférieure (308) comprenant deux parties de bord extérieures disposées a proximité des deux parois de délimitation latérales (316b), et une partie centrale sensiblement au niveau des deux parties de bord extérieures.

4. Plaque de compression du sein (300) selon la revendication 1, la paroi inférieure (308) comprenant une partie proximale disposée à proximité de la paroi de délimitation avant (316a) et une partie distale disposée à distance de la paroi de délimitation avant (316a) et la partie distale étant disposée à une distance de partie distale au-dessus du plan de référence inférieur (326).

5. Plaque de compression du sein (300) selon l'une quelconque des revendications 1 à 4, la partie de support (304) étant reliée à la paroi de délimitation arrière (316c) et comprenant une paroi de plaque arrière disposée à l'opposé de la paroi de délimitation arrière (316c) et disposée sensiblement parallèlement à la paroi de délimitation arrière (316c).

6. Plaque de compression du sein (300) selon la revendication 5, comprenant en outre une pluralité de nervures s'étendant de la paroi de délimitation arrière (316c) à la paroi de plaque arrière.

7. Plaque de compression du sein (300) selon la revendication 6, au moins une de la pluralité de nervures étant en contact avec au moins une partie de la paroi inférieure (308).

8. Plaque de compression du sein (300) selon l'une quelconque des revendications 1 à 7, au moins une partie de la paroi de transition (310) étant convexe.

9. Plaque de compression du sein (300) selon l'une quelconque des revendications 1 à 8, la paroi de délimitation avant (316a) définissant un évidement ayant une largeur supérieure disposée à proximité de la partie supérieure de la paroi de délimitation avant (316a) et une largeur inférieure disposée à proximité de la paroi inférieure (308), la largeur inférieure étant plus grande que la largeur supérieure.

10. Plaque de compression du sein (300) selon la revendication 9, l'évidement étant disposé selon un angle par rapport à la paroi de délimitation avant (316a).

11. Plaque de compression du sein (300) selon l'une quelconque des revendications 1 à 10, utilisant des contours lisses sur toutes les surfaces dans la zone d'imagerie de manière à réduire ou à empêcher de tels artefacts.

12. Plaque de compression (300) selon l'une quelconque des revendications 1, comprenant en outre des nervures qui s'étendent jusqu'à un emplacement sur la paroi de transition (310) qui est inférieur à la hauteur de la paroi de délimitation avant (316a).

13. Plaque de compression (300) selon l'une quelconque des revendications 1 à 12, comprenant une configuration ouverte en forme de bol.

14. Plaque de compression (300) selon l'une quelconque des revendications 1 à 13, la paroi de transition (310) ayant une courbure généralement lisse qui relie la paroi inférieure (308) et une surface inférieure de la partie de support (304).
